# EUROPEAN PATENT APPLICATION

(11) **EP 1 787 657 A1**
(43) Date of publication of application: **23.05.2007**
(21) Application number: 05770601.2
(22) Date of filing: 05.08.2005
(51) Int. Cl.: A61K 45/00, A61K 31/192, A61K 31/351, A61K 31/381, A61K 31/404, A61K 45/06, A61P 25/00, A61P 25/18, A61P 25/22, A61P 25/24, A61P 43/00, C07D 209/30, C07D 213/65, C07D 309/14, C07D 409/04, A61K 31/195, A61K 31/4406

(54) **THERAPEUTIC AGENT FOR PSYCHONEUROTIC DISEASE**

(30) Priority: 06.08.2004 JP 2004230228
(71) Applicant: ONO PHARMACEUTICAL CO., LTD., Osaka-shi, Osaka 541-8526 (JP)
(72) Inventor: NARITA, Masami c/o Ono Pharmaceutical Co., Ltd., Mishima-gun, Osaka 618-8585 (JP); SATO, Kazutoyo c/o Ono Pharmaceutical Co., Ltd., Mishima-gun, Osaka 618-8585 (JP)
(74) Representative: Henkel, Feiler & Hänzel
(86) International application number: PCT/JP2005/014792
(87) International publication number: WO 2006/014024

(57) **Abstract**

The present invention relates to a preventive and/or therapeutic agent for psychoneurotic diseases, comprising an EP₃ antagonist represented by the general formula (I): 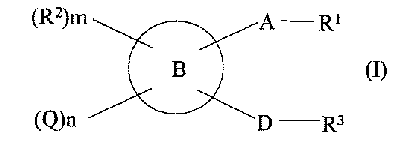 (wherein the meanings of characters are defined in the description). This compound has an antagonistic potency against EP₃ and exhibits efficacy through unusual action mechanism as a preventive and/or therapeutic agent for psychoneurotic disorder, psychosomatic disorder, anxiety disorder, depression and disorder caused by stress.

## Description

### TECHNICAL FIELD

The present invention relates to a preventive and/or therapeutic agent for psychoneurotic diseases, comprising an EP₃ antagonist.

### BACKGROUND ART

Prostaglandin E₂ (PGE₂) is known as a metabolite in the arachidonic acid cascade. It is known that PGE₂ possesses cyto-protective activity, uterine contractile activity, a pain-inducing effect, a promoting effect on digestive peristalsis, an awaking effect, a suppressive effect on gastric acid secretion, hypotensive activity, and diuretic activity.

In the recent study, it was found that PGE₂ receptor was classified into some subtypes, which play physical roles different from each other. At present, four receptor subtypes are known and they are called EP₁, EP₂, EP₃ and EP₄ respectively [J. Lipid Mediators Cell Signaling, 12, 379-391 (1995)].

The compounds descried in WO03/16254 pamphlet, WO02/16311 pamphlet and WO02/20462 pamphlet are known as an EP₃ and/or EP₄ antagonist at present.

At the same time, the use of EP₃ antagonist was reported as a therapeutic agent for pruritus (WO03/24484 pamphlet) and cosmetics which decrease the loss of scalp hair (JP-A-2002-121118); however, it has not specifically reported as a preventive and/or therapeutic agent for psychoneurotic disorder, psychosomatic disorder, anxiety disorder, depression or disorder caused by stress, *etc.*

At the same time, activation pro-hormone signaling that hypothalamus - pituitary gland - adrenal cortex system goes through is well known. In other words, an adrenocorticotropic hormone (ACTH) is produced in a pituitary gland by stimulation of an adrenocorticotropic hormone release factor (CRF) produced in a hypothalamus. Corticosterone is produced in an adrenal cortex by stimulation of this ACTH. But, excessive secretion of the corticosterone by excessive activation of this system is considered to induce a variety of a stress response, for example, a depression, anxiety disorder, psychosomatic disorder, *etc.*

At the present, for example, tricyclic antidepressant, tetracyclic antidepressant, monoamine oxidaze (MAO) inhibitor, serotonin norepinephrine reuptake inhibitor (SNRI), selective serotonin reuptake inhibitor (SSRI) and the like are used as antidepressant agents for the treatment of psychoneurotic diseases. However, the treatment effect is not enough, and it will take a long time by the time the effect often appears; sleepiness, sensation of thirst, constipation, a feeling of urination difficulty, *etc.* are seen as a side effect. Benzodiazepine class, thienodiazepine class, nonbenzodiazepine class are used for antianxiety drug. But, these treatment effect is not also enough, and a fall of a psychomotor function, loss of concentration and diminished attention, sleepiness, drift, dizziness, a headache, poor memory are seen as a side effect.

### DISCLOSURE OF THE INVENTION

An object of the present invention is to provide a preventive and/or therapeutic agent for psychoneurotic diseases which exhibits efficacy through unusual action mechanism.

The inventers have found that EP₃ antagonist is useful as a preventive and/or therapeutic agent for psychoneurotic diseases and completed the present invention.

The present invention relates to
[1.] A preventive and/or therapeutic agent for psychoneurotic disorder, which comprises an EP₃ antagonist,
[2.] The preventive and/or therapeutic agent for psychoneurotic disorder according to the above 1, wherein the psychoneurotic disorder is a disorder caused by stress,
[3.] The preventive and/or therapeutic agent for psychoneurotic disorder according to the above 1, wherein the psychoneurotic disorder is a depression, an anxiety disorder or a psychosomatic disorder,
[4.] The preventive and/or therapeutic agent for psychoneurotic disorder according to the above 1, wherein the psychoneurotic disorder is a depression, an anxiety disorder or a psychosomatic disorder attributable to stress,
[5.] The preventive and/or therapeutic agent for psychoneurotic disorder according to the above 3, wherein the depression is a depressive episode, a dysthymic disorder, a cyclothymic disorder, a bipolar emotional disorder or a manic-depression disorder,
[6.] The preventive and/or therapeutic agent for psychoneurotic disorder according to the above 3, wherein the depression shows symptoms of one or more selected from psychomotor inhibition, anxiety, impatience, idea of belittlement, suicidal ideation, headache, insomnia, generalized fatigability, feeling of fatigue, febricula, vertigo, tinnitus, stiff shoulder, dry mouth feeling, taste abnormality, palpitatio cordis, dyspnoea, low back pain, arthralgia, reproductive hypesthesia, decreased libido, menstrual disorder, dysfunctional voiding, asitia, nausea, vomiting, abdominal pain, ulcus, abdominal discomfort and coldness of limbs,
[7.] The preventive and/or therapeutic agent for psychoneurotic disorder according to the above 3, wherein the anxiety disorder is one or more selected from obsessive-compulsive disorder, panic disorder, posttraumatic stress disorder, generalized anxiety disorder, mixed anxiety depressive disorder, social avoidance and distress and anthrophobia,
[8.] The preventive and/or therapeutic agent for psychoneurotic disorder according to the above 3, wherein the psychosomatic disorder shows symptoms of one or more selected from autonomic dystonia, climacteric disorder, hypertension, hypotension, angina, asthma, hyperventilation syndrome, irritable bowel syndrome, gastric ulcer, duodenal ulcer, headache, migraine, over active bladder, enuresis, insomnia, alopecia areata, diabetes, premenstrual syndrome, dysmenorrheal, infertility, alexithymia, alcohol dependence syndrome, anorexia nervosa, bulimia nervosa, Meniere's syndrome, cervicobrachial syndrome and indefinite complaint,
[9.] The preventive and/or therapeutic agent for psychoneurotic disorder according to the above 1, which further comprises one or more selected from tricyclic antidepressant, tetracyclic antidepressant, monoamine oxidaze inhibitor, serotonin and noradrenaline reuptake inhibitor, selective serotonin reuptake inhibitor, 5-HT_{1A} receptor agonists, GABA receptor agonists, dopamine receptor antagonist, psychoanaleptic, antianxiety agent, antipsychotic agent, mitochondrial benzodiazepine receptor agonists or antagonist, NK1 antagonist, σ receptor agonists, serotonin nervous system agonists, corticotropin releasing factor receptor antagonists, proton pump inhibitors, histamine H₂-receptor antagonist, M1 receptor antagonists and EP₁ antagonist in combination,
[10.] The preventive and/or therapeutic agent for psychoneurotic disorder according to the above 1, wherein the EP₃ antagonist is a compound represented by formula (I): 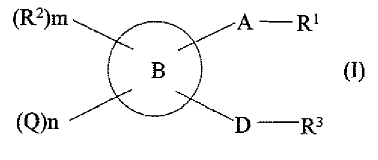 wherein all symbols have the same meanings as the described below
   or a salt thereof, a solvate thereof or a prodrug thereof,
[11.] The preventive and/or therapeutic agent for psychoneurotic disorder according to the above 10, which is
   (1) 3-(2-(((1R)-3-methyl-1-(3,5-dimethylphenyl)butyl)carbamoyl)-4-(2,5-difluorophenoxymethyl)phenyl)propanoic acid,
   (2) 3-(2-(((1R)-3-methyl-1-(3,5-dimethylphenyl)butyl)carbamoyl)-4-(2,5-dimethylphenoxymethyl)phenyl)propanoic acid,
   (3)3-(2-((((1R)-1-(3,5-dimethylphenyl)-3-methylbutyl)amino)carbonyl)-4-((2-methylphenoxy)methyl)phenyl)propanoic acid,
   (4) 3-(4-((2,5-difluorophenoxy)methyl)-2-(((4-(3,5-dimethylphenyl)tetrahydro-2H-pyran-4-yl)amino)carbonyl)phenyl)propanoic acid,
   (5) 3-(4-(3-cyanophenoxy)methyl)-2-((((1R)-1-(3,5-dimethylphenyl)-3-methylbutyl)amino)carbonyl)phenyl)propanoic acid,
   (6) 3-(4-((3-chlorophenoxy)methyl)-2-((((1R)-1-(3,5-dimethylphenyl)-3-methylbutyl)amino)carbonyl)phenyl)propanoic acid,
   (7) 3-(4-((2-chloro-5-fluorophenoxy)methyl)-2-(((4-(3,5-dimethylphenyl)tetrahydro-2H-pyran-4-yl)amino)carbonyl)phenyl)propanoic acid,
   (8) 3-(4-((2-chloro-5-methylphenoxy)methyl)-2-(((4-(3,5-dimethylphenyl)tetrahydro-2H-pyran-4-yl)amino)carbonyl)phenyl)propanoic acid,
   (9) 3-(4-((2,5-dichlorophenoxy)methyl)-2-(((4-(3,5-dimethylphenyl)tetrahydro-2H-pyran-4-yl)amino)carbonyl)phenyl)propanoic acid or
   (10) 3-(4-((2,5-difluorophenoxy)methyl)-2-(((4-(3-methylphenyl)tetrahydro-2H-pyran-4-yl)amino)carbonyl)phenyl)propanoic acid,
      a salt thereof, a solvate thereof or a prodrug thereof,
[12.] The preventive and/or therapeutic agent for psychoneurotic disorder according to the above 1, wherein the EP₃ antagonist is a compound represented by formula (II): 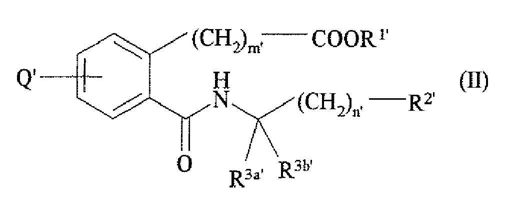 wherein all symbols have the same meanings as the described below
   or a salt thereof, a solvate thereof or a prodrug thereof,
[13.] The preventive and/or therapeutic agent for psychoneurotic disorder according to the above 12, which is
   (1) 3-(2-((((1R)-1-(3,5-dimethylphenyl)-3-methylbutyl)amino)carbonyl)-4-(5-fluoro-2-methylphenoxymethyl)phenyl)propanoic acid,
   (2) 3-(2-(((4-(benzothiophen-2-yl)tetrahydro-2H-pyran-4-yl)amino)carbonyl)-4-(2,5-difluorophenoxymethyl)phenyl)propanoic acid,
   (3) 3-(4-(2-fluoro-5-methylphenoxymethyl)-2-((((1R)-3-methyl-1-(3-methylphenyl)butyl)amino)carbonyl)phenyl)propanoic acid,
   (4) 3-(4-(2,5-difluorophenoxymethyl)-2-(((4-(2-(3-fluorophenyl)ethyl)tetrahydro-2H-pyran-4-yl)amino)carbonyl)phenyl)propanoic acid,
   (5) 3 -(4-(3,5-dimethylphenylamino)-2-(((4-(naphthalen-2-yl)tetrahydro-2H-pyran-4-yl)amino)carbonyl)phenyl)propanoic acid,
   (6) 3-(2-(((4-(benzothiophen-2-yl)tetrahydro-2H-pyran-4-yl)amino)carbonyl)-4-(3,5-dimethylphenoxy)phenyl)propanoic acid,
   (7) 3-(4-(2,5-difluorophenoxymethyl)-2-(((4-(2-phenylethyl)tetrahydro-2H-pyran-4-yl)amino)carbonyl)phenyl)propanoic acid,
   (8) 3-(2-((((1R)-1-(3,5-dimethylphenyl)-3-methylbutyl)amino)carbonyl)-4-(3-pyridyloxymethyl)carbonyl)phenyl)prapanoic acid,
   (9) 3 -(4-(2-fluoro-5-methylphenoxymethyl)-2-((((1R)-1-(3,5-dimethylphenyl)-3-methylbutyl)amino)carbonyl)phenyl)propanoic acid,
   (10) 3-(4-(3,5-dimethylphenylamino)-2-((((1R)-1-(3,5-dimethylphenyl)-3-methylbutyl)amino)carbonyl)phenyl)propanoic acid,
   (11) 3-(4-(6-fluoroindol-1-ylmethyl)-2-((((1R)-1-(3,5-dimethylphenyl)-3-methylbutyl)amino)carbonyl)phenyl)propanoic acid,
   (12) 3-(4-(3,5-dimethylphenoxy)-2-(((4-(naphthalen-2-yl)tetrahydro-2H-pyran-4-yl)amina)carbonyl)phenyl)propanoic acid,
   (13) 4-(4-(3,5-dimethylphenylamino)-2-((((1R)-1-(naphthalen-1-yl)ethyl)amino)carbonyl)phenyl)butanoic acid,
   (14) 3-(4-(2-chloro-5-methylphenoxymethyl)-2-((((1R)-3-methyl-1-(3-methylphenyl)butyl)amino)carbonyl)phenyl)propanoic acid,
   (15) 3-(4-(2,5-difluorophenoxymethyl)-2-(((4-(2-(4-fluorophenyl)ethyl)tetrahydro-2H-pyran-4-yl)amino)carbonyl)phenyl)propanoic acid,
   (16) 3-(4-(3,5-dimethylphenylamino)-2-((((1R)-1-(3-methylphenyl)-3-methylbutyl)amino)carbonyl)phenyl)propanoic acid,
   (17) 3-(4-(2-fluoro-5-methylphenoxymethyl)-2-(((4-(naphthalen-2-yl)tetrahydro-2H-pyran-4-yl)amino)carbonyl)phenyl)propanoic acid,
   (18) 3-(4-(3,5-dimethylphenoxy)-2-((((1R)-1-(3,5-dimethylphenyl)-3-methylbutyl)amino)carbonyl)phenyl)propanoic acid or
   (19) 3-(4-(3,5-dimethylphenylamino)-2-(((4-(2-(3-fluorophenyl)ethyl)tetrahydro-2H-pyran-4-yl)amino)carbonyl)phenyl)propanoic acid,
      a salt thereof, a solvate thereof or a prodrug thereof,
[14.] The preventive and/or therapeutic agent for psychoneurotic disorder according to the above 1, which comprises the EP₃ antagonist in the range of from about 1 mg to about 600 mg,
[15.] The preventive and/or therapeutic agent for psychoneurotic disorder according to the above 14, wherein the EP₃ antagonist is 3-(2-((((1R)-1-(3,5-dimethylphenyl)-3-methylbutyl)amino)carbonyl)-4-(5-fluoro-2-methylphenoxymethyl)phenyl)propanoic acid, 3-(2-(((1R)-3-methyl-1-(3,5-dimethylphenyl)butyl)carbamoyl)-4-(2,5-difluorophenoxymethyl)phenyl)propanoic acid or 3-(2-(((1R)-3-methyl-1-(3,5-dimethylphenyl)butyl)carbamoyl)-4-(2,5-dimethylphenoxymethyl)phenyl)propanoic acid,
[16.] A preventive and/or therapeutic agent for psychoneurotic disorder, which comprises 3-(2-((((1R)-1-(3,5-dimethylphenyl)-3-methylbutyl)amino)carbonyl)-4-(5-fluoro-2-methylphenoxymethyl)phenyl)propanoic acid which is administered in the range of from about 1 mg to about 600 mg per day,
[17.] The preventive and/or therapeutic agent for psychoneurotic disorder according to the above 16, wherein the psychoneurotic disorder is a depression or an anxiety disorder,
[18.] The preventive and/or therapeutic agent for psychoneurotic disorder according to the above 16, wherein it is administered in the range of from about 5 mg to about 300 mg per day,
[19.] The preventive and/or therapeutic agent for psychoneurotic disorder according to the above 18, wherein it is administered in the range of from about 10 mg to about 100 mg per day,
[20.] The preventive and/or therapeutic agent for psychoneurotic disorder according to the above 19, wherein it is administered in the range of from about 10 mg to about 50 mg per day,
[21.] The preventive and/or therapeutic agent for psychoneurotic disorder according to the above 16, wherein the 3-(2-((((1R)-1-(3,5-dimethylphenyl)-3-methylbutyl)amino)carbonyl)-4-(5-fluoro-2-methylphenoxymethyl)phenyl)propanoic acid is contained in the range of from about 1 mg to about 100 mg per solid preparation,
[22.] The preventive and/or therapeutic agent for psychoneurotic disorder according to the above 21, wherein it is contained in the range of from about 2.5 mg to about 25 mg per solid preparation,
[23.] A method for preventing and/or treating psychoneurotic disorder, which comprises administering an effective dose of the compound of formula (I) or formula (II), a salt thereof, a solvate thereof or a prodrug thereof to a mammal,
[24.] The method according to the above 23, wherein the compound of formula (II) is 3-(2-((((1R)-1-(3,5-dimethylphenyl)-3-methylbutyl)amino)carbonyl)-4-(5-fluoro-2-methylphenoxymethyl)phenyl)propanoic acid is administered in the range of from about 1 mg to about 600 mg per day,
[25.] Use of a compound of formula (I) or (II), a salt thereof, a solvate thereof or a prodrug thereof, for the manufacture of a preventive and/or therapeutic agent for psychoneurotic disorder, and
[26.] The use according to the above 25, wherein the compound of formula (II) is 3-(2-((((1R)-1-(3,5-dimethylphenyl)-3-methylbutyl)amino)carbonyl)-4-(5-fluoro-2-methylphenoxymethyl)phenyl)propanoic acid and is administered in the range of from about 1 mg to about 600 mg per day.

In the present invention, the EP₃ antagonist may be any compound, so long as it has an antagonistic potency against EP₃, and it is not limited by presence or absence and strength or weakness of an antagonistic potency against other EP.

In the present specification, the EP₃ antagonist includes a compound represented by formula (I): 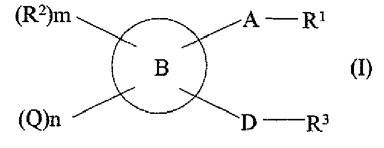 wherein R¹ is -COOH, -COOR⁴, -CH₂OH, -CONR⁵SO₂R⁶, -CONR⁷R⁸, -CH₂NR⁵SO₂R⁶, -CH₂NR⁹COR¹⁰, -CH₂NR⁹CONR⁵SO₂R⁶, -CH₂SO₂NR⁹COR¹⁰, -CH₂OCONR⁵SO₂R⁶, tetrazole, 1,2,4-oxadiazol-5-one, 1,2,4-oxadiazol-5-thione, 1,2,4-thiadiazol-5-one, 1,3-thiazolidin-2,4-dione or 1,2,3,5-oxathiadiazol-2-one;
R⁴ is C1-6 alkyl or -(C1-4 alkylene)-R¹¹;
R¹¹ is hydroxy, C1-4 alkoxy, -COOH, C1-4 alkoxycarbonyl or -CONk⁷R⁸;
R⁵ is hydrogen or C 1-6 alkyl;
R⁶ is
(i) C1-6 alkyl,
(ii) a C3-15 mono-, bi- or tri-carbocyclic ring or a 3- to 15-membered mono-, bi- or tri-heterocyclic ring which is substituted with 1-5 of R¹² or unsubstituted,
(iii) C1-6 alkyl, C2-6 alkenyl or C2-6 alkynyl substituted with a C3-15 mono-, bi- or tri-carbocyclic ring or a 3- to 15-membered mono-, bi- or tri-heterocyclic ring which is substituted with 1-5 of R¹² or unsubstituted;
   R⁷ and R⁸ each independently, is

(i) hydrogen,
(ii) C1-6 alkyl,
(iii) hydroxy,
(iv) -COR¹⁷,
(v) a C3-15 mono-, bi- or tri-carbocyclic ring or a 3- to 15-membered mono-, bi- or tri-heterocyclic ring which is substituted with 1-5 of R¹² or unsubstituted, or
(vi) C1-4 alkyl substituted with a C3-15 mono-, bi- or tri-carbocyclic ring or a 3-to 15-membered mono-, bi- or tri-heterocyclic ring which is substituted with 1-5 of R¹² or unsubstituted;
   R⁹ is hydrogen or C1-6 alkyl;
   R¹⁰ is

(i) hydrogen,
(ii) C1-6 alkyl,
(iii) a C3-15 mono-, bi- or tri-carbocyclic ring or a 3- to 15-membered mono-, bi- or tri-heterocyclic ring which is substituted with 1-5 of R¹² or unsubstituted, or
(iv) C1-6 alkyl, C2-6 alkenyl or C2-6 alkynyl substituted with a C3-15 mono-, bi- or tri-carbocyclic ring or a 3- to 15-membered mono-, bi- or tri-heterocyclic ring which is substituted with 1-5 of R¹² or unsubstituted;
   R¹² is (a) C1-6 alkyl, (b) C1-6 alkoxy, (c) C1-6 alkylthio, (d) halogen, (e) CF₃, (f) cyano, (g) nitro, (h) hydroxy, (i) -COOR¹³, (j) -NHCOR¹³, (k) -SO₂R¹⁴, (1) -NR¹⁵R¹⁶, (m) a C3-7 mono-carbocyclic ring which is substituted with C1-4 alkyl or oxo or unsubstituted, (n) a 3- to 7-membered mono-heterocyclic ring which is substituted with C1-4 alkyl or oxo or unsubstituted, or (o) C1-4 alkyl substituted with hydroxy, -COOR¹³, -NHCOR¹³, -SO₂R¹⁴ or -NR¹⁵R¹⁶;
   R¹³ is hydrogen, C1-4 alkyl, phenyl, or phenyl-(C1-4) alkyl;
   R¹⁴ is C1-4 alkyl;
   R¹⁵ and R¹⁶ each independently, is hydrogen, C1-4 alkyl, phenyl, or phenyl-(C1-4) alkyl;
   R¹⁷ is C1-4 alkyl or phenyl;
   A is

(i) a single bond,
(ii) C1-6 alkylene,
(iii) C2-6 alkenylene,
(iv) C2-6 alkynylene,
(v) -O-(C1-3 alkylene),
(vi) -S-(C1-3 alkylene),
(vii) -NR²⁰-(C1-3 alkylene),
(viii) -CONR²¹-(C1-3 alkylene),
(ix) -(C1-3 alkylene)-O-(C1-3 alkylene),
(x) -(C1-3 alkylene)-S-(C1-3 alkylene),
(xi) -(C1-3 alkylene)-NR²⁰-(C1-3 alkylene),
(xii) -(C1-3 alkylene)-CONR²¹-(C1-3 alkylene),
(xiii) -Cyc1,
(xiv) -(C1-4 alkylene)-Cyc1 or
(xv) -Cyc1-(C1-4 alkylene);
   the alkylene, alkenylene and alkynylene in A may be substituted with 1-6 of the following substituents of (a)-(i):
   (a) C1-6 alkyl, (b) C1-6 alkoxy, (c) halogen, (d) CHF₂, (e) CF₃, (f) OCHF₂, (g) OCF₃, (h) hydroxy, (i) hydroxy-(C1-4) alkyl;
      R²⁰ is hydrogen, C1-4 alkyl, -SO₂-(C1-4) alkyl or C2-5 acyl;
      R²¹ is hydrogen or C 1-4 alkyl;
      Cyc1 is a C3-7 mono-carbocyclic ring or a 3- to 7-membered mono-heterocyclic ring which is substituted with 1-4 of C1-6 alkyl, C1-6 alkoxy, C1-6 alkylthio, C2-6 alkenyl, C2-6 alkynyl, halogen, CHF₂, CF₃, nitro or cyano, or unsubstituted;
      B ring is a C3-12 mono- or bi-carbocyclic ring or a 3- to 12-membered mono- or bi-heterocyclic ring;
      R² is C1-6 alkyl, C1-6 alkoxy, C1-6 alkylthio, C2-6 alkenyl, C2-6 alkynyl, halogen, CHF₂, CF₃, nitro, cyano, phenyl or oxo;
      m is 0, 1 or 2;
      n is 1 or 2 when -D-R³ binds to B ring at the ortho position based on -A-R¹;
      n is 0, 1 or 2 when -D-R³ binds to B ring at the non-ortho position based on -A-R¹;
      Q is

(1)
   (i) -(C1-4 alkylene, C2-4 alkenylene or C2-4 alkynylene)-Cyc2,
   (ii) -(C1-4 alkylene)-Z-Cyc3,
   (iii) C1-4 alkyl substituted with a substituent(s) selected from -NR²⁴R²⁵, -S(O)ₚR²⁵, cyano, -NR²³COR²⁷, -NR²³SO₂R²⁸ and -NR²³CONR²⁴R²⁵,
   (iv) C1-4 alkoxy(C1-4) alkoxy, -NR²³COR²⁷, -COR²⁸, -OSO₂R²⁸, -NR²³SO₂R²⁸ or -NR²³CONR²⁴R²⁵,
   (v) a C3-7 mono-carbocyclic ring or a 3- to 6-membered mono-heterocyclic ring which is substituted with 1-5 of R³⁰, wherein one R³⁰ of them always binds to the ring at the non 1-position,
   (vi) a C8-15 mono-, bi- or tri-carhocyclic ring or a 7- to 15-membered mono-, bi- or tri-heterocyclic ring which is substituted with 1-5 of R³⁰ or unsubstituted,
   (vii) -T-Cyc5,
   (viii) -L-Cyc6-1, -L-(C3-6 cycloalkyl), -L-CH₂-(C3-6 cycloalkyl), -L-(C2-4 alkylene)-Cyc6-2 or -L-(C1-4 alkylene)_{q}-Cyc6-3, wherein the cycloalkyl is substituted with 1-5 of R³⁰ or unsubstituted,
(2)
   (i) phenoxy,
   (ii) benzyloxy,
   (iii) hydroxy(C1-4) alkyl,
   (iv) C1-4 alkoxy(C1-4) alkyl, or
   (v) -(C1-4 alkylene)-O-benzyl, or
(3)
   (i) C2-6 alkenyl,
   (ii) C2-6 alkynyl,
   (iii) C1-6 alkyl substituted with 1-3 halogen(s),
   (iv) cyano,
   (v) nitro,
   (vi) -NR³³R³⁴,
   (vii) -CONR³³R³⁴,
   (viii) -S(O)ₚ-(C1-4) alkynyl,
   (ix) -S(O)ₚ-CHF₂,
   (x) -S(O)ₚ-NR³³R³⁴,
   (xi) -O-(C3-6) alkynyl,
   (xii) -O-CHF₂, or
   (xiii) C3-7 cycloalkyl;
      R²² is hydrogen, C1-4 alkyl, -SO₂-(C1-4) alkyl or C2-5 acyl;
      R²³ is hydrogen, C1-4 alkyl, phenyl or phenyl(C1-4) alkyl;
      R²⁴ and R²⁵ each independently, is hydrogen, C1-4 alkyl, Cyc4 or (C1-4 alkylene)-Cyc4;
      R²⁶ is C1-4 alkyl or Cyc4;
      R²⁷ is hydrogen, C1-4 alkyl, -OR²⁹ or Cyc4;
      R²⁸ is C1-4 alkyl, Cyc4 or -(C1-4 alkylene)-Cyc4;
      R²⁹ is hydrogen, C1-4 alkyl, Cyc4 or (C1-4 alkylene)-Cyc4;
      R³⁰ is C1-8 alkyl, C1-8 alkoxy, C1-8 alkylthio, halogen, CF₃, OCF₃, SCF₃, CRF₂, OCHF₂, SCHF₂, hydroxy, cyano, nitro, -NR³¹R³², -CONR³¹R³², formyl, C2-5 acyl, hydroxy(C1-4) alkyl, C1-4 alkoxy(C1-4) alkyl, C1-4 alkylthio(C1-4) alkyl, -(C1-4 alkylene)-CONR³¹R³², -SO₂(C1-4) alkyl, -NR²³CO-(C1-4) alkyl, -NR²³SO₂-(C1-4) alkyl, benzoyl, oxo, a C3-7 mono-carbocyclic ring, a 3- to 7-membered mono-heterocyclic ring, -(C1-4 alkylene)-NR³¹R³², -M-(C3-7 mono-carbocyclic ring), or -M-(3- to 7-membered mono-heterocyclic ring);
      the C3-7 mono-carbocyclic ring and 3- to 7-membered mono-heterocyclic ring in R³⁰ may be substituted with 1-5 of the following substituents (a)-(I):
      (a) C1-6 alkyl, (b) C2-6 alkenyl, (c) C2-6 alkynyl, (d) C1-6 alkoxy, (e) C1-6 alkylthio, (f) halogen, (g) CHF₂, (h) CF₃, (I) nitro, (j) cyano, (k) hydroxy, (I) amino;
         M is -O-, -S-, C1-4 alkylene, -O-(C1-4 alkylene)-, -S-(C1-C4 alkylene)-,
      -(C1-4 alkylene)-O- or -(C1-4 alkylene)-S-;
      R³¹ and R³² each independently, is hydrogen or C1-4 alkyl,
      Cyc2 is a C3-15 mono-, bi- tri-carbocyclic ring or a 3- to 15-membered mono-, bi- tri-heterocyclic ring which is substituted with 1-5 of R³⁰ or unsubstituted;
      Z is -O-, -S(O)ₚ-, -NR²²- -NR²³CO-, -NR²³SO₂-, -NR²²-(C1-4 alkylene)-, -S(O)ₚ-(C1-4 alkylene)-, -O-(C2-4 alkylene)-, -NR²³CO-(C1-4 alkylene) or -NR²³SO₂(C1-4 alkylene);
      p is 0, 1 or 2;
      Cyc3 is a C3-15 mono-, bi- tri-carbocyclic ring or a 3- to 15-membered mono-, bi- tri-heterocyclic ring which is substituted with 1-5 of R³⁰ or unsubstituted;
      Cyc4 is a C3-12 mono-, bi-carbocyclic ring or a 3- to 12-membered mono-, bi-heterocyclic ring which is substituted with 1-5 of R³⁰ or unsubstituted;
      T is -O-, -NR²²-, -O-(C1-4 alkylene)-, -S(O)ₚ-(C1-4 alkylene)- or -NR²²-(C1-4 alkylene)-;
      Cyc5 is a 3- to 15-membered mono-, bi- tri-heterocyclic ring which is substituted with 1-5 of R³⁰ or unsubstituted;
      q is 0 or 1;
      L is -O- or -NR²³-;
      Cyc6-1 is phenyl or benzyl which is substituted with one or more R³⁰;
      Cyc6-2 is a C3-6 mono-carbocyclic ring which is substituted with 1-5 of R³⁰ or unsubstituted;
      Cyc6-3 is a C7-15 mono-, bi- or tri-carbocyclic ring which is substituted with 1-5 of R³⁰ or unsubstituted;
      R³³ and R³⁴ each independently, is hydrogen, C1-4 alkyl, phenyl or benzyl, or
      NR³³R³⁴ is a 3- to 6-membered mono-heterocyclic ring containing one nitrogen and optionally containing one hetero atom selected from nitrogen, oxygen and sulfur;
      D is

(1) a 1- or 2-membered linking chain comprising an atom(s) selected from carbon, nitrogen, oxygen and sulfur, which may contain a double bond or a triple bond in the chain and may be substituted with 1-4 of R⁴⁰,
(2) a 3- to 6-membered linking chain comprising atoms selected from carbon, nitrogen, oxygen and sulfur, which may contain a double bond or a triple bond in the chain and may be substituted with 1-12 of R⁴⁰, wherein R⁴⁰ substituted on the atom bound to R³, and R⁴² which is a substituent of R³, taken together may form -(CH₂)_{y}-, in which y is 1-4, or
(3) a 7- to 10-membered linking chain comprising atoms selected from carbon, nitrogen, oxygen and sulfur, which may contain a double bond or a triple bond and may be substituted with 1-20 of R⁴⁰, wherein R⁴⁰ substituted on the atom bound to R³, and R⁴² which is a substituent of R³, taken together may form -(CH₂)_{y}-;
   R⁴⁰ is (a) C1-8 alkyl, (b) C2-8 alkenyl, (c) C2-8 alkynyl, (d) oxo, (e) halogen, (f) CF₃, (g) hydroxy, (h) C1-6 alkoxy, (i) C2-6 alkenyloxy, (j) C2-6 alkynyloxy, (k) OCF₃, (1) -S(O)ₚ-(C1-6) alkyl, (m) -S(O)ₚ-(C2-6) alkenyl, (n) -S(O)ₚ-(C2-6) alkynyl, (o) C2-5 acyl, (p) Cyc9, (q) C1-4 alkaxy(C1-4) alkoxy, or (r) C1-8 alkyl, C2-8 alkenyl or C2-8 alkynyl substituted with 1 or 2 of substituents selected from halogen, CF₃, OCF₃, hydroxy, cyano, C1-4 alkoxy, -S(O)ₚ-(C1-6) alkyl, Cyc9 and C1-4 alkoxy(C1-4) alkoxy or
   two R⁴⁰'s taken together with the atom of a linking chain to which they bind, may form a C3-15 mono-, bi- or tri-carbocyclic ring or a 3- to 15-membered mono-, bi-or tri-heterocyclic ring containing 1-2 hetero atom(s) selected from O, S, SO₂ and N, wherein the carbocyclic ring and the heterocyclic ring may be substituted with 1-3 substituent(s) selected from C1-4 alkyl, C1-4 alkoxy, C2-5 acyl, SO₂(C1-4 alkyl), phenyl and phenyl(C1-4) alkyl;
   Cyc9 is a C3-6 mono-carbocyclic ring or a 3- to 6-membered mono-heterocyclic ring, which is substituted with 1-5 of R⁴¹ or unsubstituted;
   R⁴¹ is C1-4 alkyl, C1-4 alkoxy, C1-4 alkylthio, C1-4 alkoxy(C1-4) alkyl, halogen, CF₃, OCF₃, SCF₃, hydroxy, cyano, formyl, C2-5 acyl, -SO₂-(C1-4) alkyl, -NR²³CO-(C1-4) alkyl, benzoyl or oxo;
   R³ is

(1) C1-6 alkyl or
(2) a C3-15 mono-, bi- or tri-carbocyclic ring or a 3- to 15-membered mono-, bi- or tri-heterocyclic ring which is substituted with 1-5 of R⁴² or unsubstituted;
   R⁴² is (a) C1-6 alkyl, (b) C1-6 alkoxy, (c) C1-6 alkylthio, (d) halogen, (e) cyano, (f) CF₃, (g) CHF₂, (h) OCF₃, (i) OCHF₂, (j) SCF₃, (k) -NR⁴³R⁴⁴, (1) -SO₂R⁴⁵, (m) -NR⁴⁶COR⁴⁷, (n) hydroxy, (o) oxo, (p) C1-4 alkoxy(C1-4) alkyl, (q) Cyc10, (r) C1-6 alkylene-Cyc10, (s) -CO-Cyc10, (t) -W-Cyc10, (u) -(C1-6 alkylene)-W-Cyc10, (v) -W-(C 1-6 alkylene)-Cyc10 or (w) -(C1-6 alkylene)-W-(C1-6 alkylene)-Cyc10;
   R⁴³ and R⁴⁴ each independently, is hydrogen or C1-4 alkyl;
   R⁴⁵ is C1-4 alkyl;
   R⁴⁶ is hydrogen or C1-4 alkyl;
   R⁴⁷ is hydrogen or C1-4 alkyl;
   Cyc10 is a C3-12 mono- or bi-carbocyclic ring or a 3- to 12-membered mono- or bi-heterocyclic ring which is substituted with 1-5 of substitutes of the following (a)-(j) or unsubstituted,
   (a) C1-4 alkyl, (b) C2-5 acyl, (c) 1-4 alkoxy, (d) halogen, (e) hydroxy, (f) nitro, (g) cyano, (h) amine, (i) CF₃, (j) OCF₃;
      W is -O-, -S(O)ₚ- or -NR⁴⁸-;
      R⁴⁸ is hydrogen or C1-4 alkyl;
      (Hereinafter, which is abbreviated to compound IA.)
      or a salt thereof, a solvate thereof or a prodrug thereof.

Furthermore, a preferably compound of formula (I) is the compound IA wherein,
in the compound of formula (I),
n is 1 or 2;
Q is
(1)
   (i) -(C1-4 alkylene, C2-4 alkenylene or C2-4 alkynylene)-Cyc2,
   (ii) -(C1-4 alkylene)-Z-Cyc3,
   (iii) C1-4 alkyl substituted with a substituent(s) selected from -NR²⁴R²⁵, -S(O)ₚR²⁵, cyano, -NR²³COR²⁷, -NR²³SO₂R²⁸ and -NR²³CONR²⁴R²⁵,
   (iv) C1-4 alkoxy(C1-4) alkoxy, -NR²³COR²⁷, -COR²⁸, -OSO₂R²⁸, -NR²³SO₂R²⁸ or -NR²³CONR²⁴R²⁵,
   (v) a C3-7 mono-carbocyclic ring or a 3- to 6-membered mono-heterocyclic ring which is substituted with 1-5 of R³⁰, wherein one R³⁰ of them always binds to the ring at the non 1-position,
   (vi) a C8-15 mono-, bi- or tri-carbocyclic ring or a 7- to 15-membered mono-, bi- or tri-heterocyclic ring which is substituted with 1-5 of R³⁰ or unsubstituted,
   (vii) -T-Cyc5,
   (viii) -L-Cyc6-1, -L-(C2-4 alkylene)-Cyc6-2 or-L-(C1-4 alkylene)_{q}-Cye6-3;
      D is

(1) a 1- or 2-membered linking chain comprising an atom(s) selected from carbon, nitrogen, oxygen and sulfur, which may contain a double bond or a triple bond in the chain and may be substituted with 1-4 of R⁴⁰,
(2) a 3- to 6-membered linking chain comprising atoms selected from carbon, nitrogen, oxygen and sulfur, which may contain a double bond or a triple bond in the chain and may be substituted with 1-12 of R⁴⁰, wherein R⁴⁰ substituted on the atom bound to R³, and R⁴² which is a substituent of R³, taken together may form -(CH₂)_{y}-;
   in the compound of formula (I),
   n is 1 or 2;
   Q is
(2)
   (i) phenoxy,
   (ii) benzyloxy,
   (iii) hydroxy(C1-4) alkyl,
   (iv) C1-4 alkoxy(C1-4) alkyl, or
   (v) -(C1-4 alkylene)-O-(C1-4 alkylene)-Cyc7;
      D is
(2) a 3- to 6-membered linking chain comprising atoms selected from carbon, nitrogen, oxygen and sulfur, which may contain a double bond or a triple bond in the chain and may be substituted with 1-12 of R⁴⁰, wherein R⁴⁰ substituted on the atom bound to R³, and R⁴² which is a substituent of R³, taken together may form -(CH₂)_{y}-;
   in the compound of formula (I),
   n is 1 or 2;
   Q is
(3)
   (i) C2-6 alkenyl,
   (ii) C2-6 alkynyl,
   (iii) C1-6 alkyl substituted with 1-3 halogen(s),
   (iv) cyano,
   (v) nitro,
   (vi) -NR³³R³⁴,
   (vii) -CONR³³R³⁴,
   (viii) -S(O)ₚ-(C1-4) alkynyl,
   (ix) -S(O)ₚ-CHF₂,
   (x) -S(O)ₚ-NR³³R³⁴,
   (xi) -O-(C3-6) alkynyl,
   (xii) -O-CHF₂, or
   (xiii) C3-7 cycloalkyl;
      D is

(1) a 1- or 2-membered linking chain comprising an atom(s) selected from carbon, nitrogen, oxygen and sulfur, which may contain a double bond or a triple bond in the chain and may be substituted with 1-4 of R⁴⁰;
   in the compound of formula (I),
   n is 0;
   D is

(1) a 1- or 2-membered linking chain comprising an atom(s) selected from carbon, nitrogen, oxygen and sulfur, which may contain a double bond or a triple bond in the chain and may be substituted with 1-4 of R⁴⁰,
(2) a 3- to 6-membered linking chain comprising atoms selected from carbon, nitrogen, oxygen and sulfur, which may contain a double bond or a triple bond in the chain and may be substituted with 1-12 of R⁴⁰, wherein R⁴⁰ substituted on the atom bound to R³, and R⁴² which is a substituent of R³, taken together may form -(CH₂)_{y}-;
   and in the compound of formula (I), n is 0, 1 or 2;
   Q is

(1)
   (i) -(C1-4 alkylene, C2-4 alkenylene or C2-4 alkynylene)-Cyc2,
   (ii) -(C1-4 alkylene)-Z-Cyc3,
   (iii) C1-4 alkyl substituted with a substituent(s) selected from -NR²⁴R²⁵, -S(O)ₚR²⁵, cyano, -NR²³COR²⁷, -NR²³SO₂R²⁸ and -NR²³CONR²⁴R²⁵,
   (iv) C1-4 alkoxy(C1-4) alkoxy, -NR²³COR²⁷, -COR²⁸, -OSO₂R²⁸, -NR²³SO₂R²⁸ or -NR²³CONR²⁴R²⁵,
   (v) a C3-7 mono-carbocyclic ring or a 3- to 6-membered mono-heterocyclic ring which is substituted with 1-5 of R³⁰, wherein one R³⁰ of them always binds to the ring at the non 1-position,
   (vi) a C8-15 mono-, bi- or tri-carbocyclic ring or a 7- to 15-membered mono-, bi- or tri-heterocyclic ring which is substituted with 1-5 of R³⁰ or unsubstituted,
   (vii) -T-Cyc5,
   (viii) -L-Cyc6-1, -L-(C2-4 alkylene)-Cyc6-2 or -L-(C1-4 alkylene)_{q}-Cyc6-3,
(2)
   (i) phenoxy,
   (ii) benzyloxy,
   (iii) hydroxy(C1-4) alkyl,
   (iv) C1-4 alkoxy(C1-4) alkyl, or
   (v) -(C1-4 alkylene)-O-(C 1-4 alkylene)-Cyc7, or
(3)
   (i) C2-6 alkenyl,
   (ii) C2-6 alkynyl,
   (iii) C1-6 alkyl substituted with 1-3 halogen(s),
   (iv) cyano,
   (v) nitro,
   (vi) -NR³³R³⁴,
   (vii) -CONR³³R³⁴,
   (viii) -S(O)ₚ-(C1-4) alkynyl,
   (ix) -S(O)ₚ-CHF₂,
   (x) -S(O)ₚ-NR³³R³⁴,
   (xi) -O-(C3-6) alkynyl,
   (xii) -O-CHF₂, or
   (xiii) C3-7 cycloalkyl;
      D is
(3) a 7- to 10-membered linking chain comprising atoms selected from carbon, nitrogen, oxygen and sulfur, which may contain a double bond or a triple bond and may be substituted with 1-20 of R⁴⁰, wherein R⁴⁰ substituted on the atom bound to R³, and R⁴² which is a substituent of R³, taken together may form -(CH₂)_{y}-.

Furthermore, in the preferable compound described above, and more preferable compounds include compounds wherein
D is
(1) a 1- or 2-membered linking chain comprising an atom(s) selected from carbon, nitrogen, oxygen and sulfur, which may contain a double bond or a triple bond in the chain and may be substituted with 1-4 of R⁴⁰; or
   D is
(2) a 3- to 6-membered linking chain comprising atoms selected from carbon, nitrogen, oxygen and sulfur, which may contain a double bond or a triple bond in the chain and may be substituted with 1-12 of R⁴⁰, wherein R⁴⁰ substituted on the atom bound to R³, and R⁴² which is a substituent of R³, taken together may form -(CH₂)y-.

The combination of the preferable substituents in the compound represented by the formula (I) described above is followed by the combination of the preferable substituents described in WO03/016254 pamphlet.

In the compounds represented by the formula (I) described above, the compounds described in Examples of WO03/016254 pamphlet is especially preferable, and among these, more preferred compounds include:
(1) 3-(2-(((1R)-3-methyl-1-(3,5-dimethylphenyl)butyl)carbamoyl)-4-(2,5-difluorophenoxymethyl)phenyl)propanoic acid (hereinafter, which may be abbreviated to compound (II).),
(2) 3-(2-(((1R)-3-methyl-1-(3,5-dimethylphenyl)butyl)carbamoyl)-4-(2,5-dimethylphenoxymethyl)phenyl)propanoic acid (hereinafter, which may be abbreviated to compound (III).),
(3) 3-(2-((((1R)-1-(3,5-dimethylphenyl)-3-methylbutyl)amino)carbonyl)-4-((2-methylphenoxy)methyl)phenyl)propanoic acid,
(4) 3-(4-((2,5-difluorophenoxy)methyl)-2-(((4-(3,5-dimethylphenyl)tetrahydro-2H-pyran-4-yl)amino)carbonyl)phenyl)propanoic acid,
(5) 3-(4-(3-cyanophenoxy)methyl)-2-((((1R)-1-(3,5-dimethylphenyl)-3-methylbutyl)amino)carbonyl)phenyl)propanoic acid,
(6) 3-(4-((3-chlorophenoxy)methyl)-2-((((1R)-1-(3,5-dimethylphenyl)-3-methylbutyl)amino)carbonyl)phenyl)propanoic acid,
(7) 3-(4-((2-chloro-5-fluorophenoxy)methyl)-2-(((4-(3,5-dimethylphenyl)tetrahydro-2H-pyran-4-yl)an 3zno)carbonyl)phenyl)propanoic acid,
(8) 3-(4-((2-chloro-5-methylphenoxy)methyl)-2-(((4-(3,5-dimethylphenyl)tetrahydro-2H-pyran-4-yl)amino)carbonyl)phenyl)propanoic acid,
(9) 3-(4-((2,5-dichlorophenoxy)methyl)-2-(((4-(3,5-dimethylphenyl)tetrahydro-2H-pyran-4-yl)amino)carbonyl)phenyl)propanoic acid or
(10) 3-(4-((2,5-difluorophenoxy)methyl)-2-(((4-(3-methylphenyl)tetrahydro-2H-pyran-4-yl)amino)carbonyl)phenyl)propanoic acid.

In the present specification, the definition of terms of groups and substituents represented by the compound of formula (I) are followed by the definition of terms described in WO03/016254 pamphlet.

In the present specification, the EP₃ antagonist includes a compound represented by formula (II): 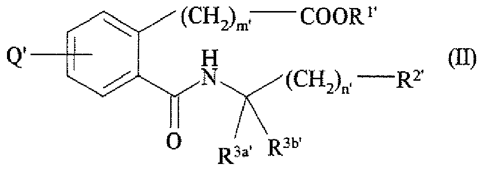 wherein R^{1'} is hydrogen or C1-4 alkyl;
R^{2'} is phenyl, naphthyl, benzofuranyl or benzothionyl, which is unsubstituted or substituted with 1-2 of C1-4 alkyl and halogen;
Q' is (i) -CH₂-O-Cyc1', (ii) -CH₂-Cyc2' or (iii) -L'-Cyc3';
Cyc1' is phenyl or pyridyl, which is unsubstituted or substituted with 1-2 of R^{4'};
Cyc2' is indolyl which is unsubstituted or substituted with 1-2 of R^{4'};
Cyc3' is phenyl substituted with 1-2 of R^{4'};
L' is -O- or -NH-;
R^{3a'} and R^{3b'} each independently is hydrogen or C1-4 alkyl or
R^{3a'} and R^{3b'}, taken together with the carbon atom to which they are attached, form tetrahydro-2H-pyran;
m' is 2 or 3;
n' is 0, 1 or 2;
R^{4'} is C1-4 alkyl, C1-4 alkylthio, halogen or cyano, or when Cyc3' is phenyl substituted with two R^{4'}, two R^{4'} taken together with phenyl may form 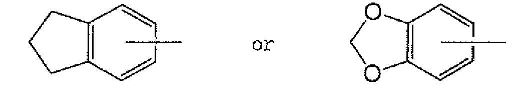 a salt thereof, a solvate thereof or a prodrug thereof

In the present specification, the definition of group and substituent represented by the compound of formula (II) is as follows.

C1-4 alkyl includes methyl, ethyl, propyl, butyl and isomers thereof.

C1-4 alkylthio includes methythio, ethylthio, propylthio, butylthio and isomers thereof.

The halogen includes fluoride, chloride, bromide and iodide.

In the present invention, preferable R^{1'} is hydrogen, methyl or ethyl.

In the present invention, preferable n' is 0 or 2.

In the present invention, a preferable substituent of ring represented by R^{2'} is methyl or fluoride.

In the present invention, preferable R^{3a'} and R^{3b'} each independently is hydrogen, methyl, isobutyl, or tetrahydro-2H-pyran which represented by R^{3a'} and R^{3b'} with the carbon atom to which they are attached. More specifically, as a group 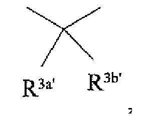 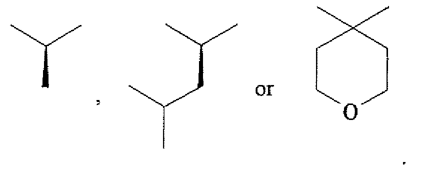 is a preferable group.

In the present invention, preferable R^{4'} is methyl, thiomethyl, fluoride, chloride or cyano.

The EP₃ antagonist represented by the compound of formula (II) is preferably,
(1) 3-(2-((((1R)-1-(3,5-dimethylphenyl)-3-methylbutyl)amino)carbonyl)-4-(5-fluoro-2-methylphenoxymethyl)phenyl)propanoic acid (hereinafter, which may be abbreviated to compound (I).),
(2) 3-(2-(((4-(benzothlophen-2-yl)tetrahydro-2H-pyran-4-yl)amino)carbonyl)-4-(2,5-difluorophenoxymethyl)phenyl)propanoic acid,
(3) 3-(4-(2-fluoro-5-methylphenoxymethyl)-2-((((1R)-3-methyl-1-(3-methylphenyl)butyl)amino)carbonyl)phenyl)propanoic acid,
(4) 3-(4-(2,5-difluorophenoxymethyl)-2-(((4-(2-(3-fluorophenyl)ethyl)tetrahydro-2H-pyran-4-yl)amino)carbonyl)phenyl)propanoic acid,
(5) 3-(4-(3,5-dimethylphenylamino)-2-(((4-(naphthalen-2-yl)tetrahydro-2H-pyran-4-yl)amino)carbonyl)phenyl)propanoic acid,
(6) 3-(2-(((4-(benzothlophen-2-yl)tetrahydro-2H-pyran-4-yl)amino)carbonyl)-4-(3,5-dimethylphenoxy)phenyl)propanoic acid,
(7) 3-(4-(2,5-difluorophenoxymethyl)-2-(((4-(2-phenylethyl)tetrahydro-2H-pyran-4-yl)amino)carbonyl)phenyl)propanoic acid,
(8) 3-(2-((((1R)-1-(3,5-dimethylphenyl)-3-methylbutyl)amino)carbonyl)-4-(3-pyridyloxymethyl)carbonyl)phenyl)propanoic acid,
(9) 3-(4-(2-fluoro-5-methylphenoxymethyl)-2-((((1R)-1-(3,5-dimethylphenyl)-3-methylbutyl)amino)carbonyl)phenyl)propanoic acid,
(10) 3-(4-(3,5-dimethylphenylamino)-2-((((1R)-1-(3,5-dimethylphenyl)-3 methylbutyl)amino)carbonyl)phenyl)propanoic acid,
(11) 3-(4-(6-fluoroindol-1-ylmethyl)-2-((((1R)-1-(3,5-dimethylphenyl)-3-methylbutyl)amino)earbonyl)phenyl)propanoic acid,
(12) 3-(4-(3,5-dimethylphenoxy)-2-(((4-(naphthalen-2-yl)tetrahydro-2H-pyran-4-yl)amino)carbonyl)phenyl)propanoic acid,
(13) 4-(4-(3,5-dimethylphenylamino)-2-((((1R)-1-(naphthalen-1-yl)ethyl)amino)carbonyl)phenyl)butanoic acid,
(14) 3-(4-(2-chloro-5-methylphenoxymethyl)-2-((((1R)-3-methyl-1-(3-methylphenyl)butyl)amino)carbonyl)phenyl)propanoic acid,
(15) 3-(4-(2,5-difluorophenoxymethyl)-2-(((4-(2-(4-fluorophenyl)ethyl)tetrahydro-2H-pyran-4-yl)amino)carbonyl)phenyl)propanoic acid,
(16) 3-(4-(3,5-dimethylphenylamino)-2-((((1R)-1-(3-methylphenyl)-3-methylbutyl)amino)carbonyl)phenyl)propanoic acid,
(17) 3-(4-(2-fluoro-5-methylphenoxymethyl)-2-(((4-(naphthalen-2-yl)tetrahydro-2H-pyran-4-yl)amino)carbonyl)phenyl)propanoic acid,
(18) 3-(4-(3,5-dimethylphenoxy)-2-((((1R)-1-(3,5-dimethylphenyl)-3-methylbutyl)amino)carbonyl)phenyl)propanoic acid or
(19) 3-(4-(3,5-dimethylphenylamino)-2-(((4-(2-(3-fluorophenyl)ethyl)tetrahydro-2H-pyran-4-yl)amino)carbonyl)phenyl)propanoic acid,
   a salt thereof a solvate thereof or a prodrug thereof.

In the present specification, the EP₃ antagonist includes the compound which has an antagonistic potency against EP₃ described in WO99/47497 pamphlet, WO00/20371 pamphlet, WO01/62708 pamphlet, WO02/16311 pamphlet and WO02/20462 pamphlet.

In the present specification, the EP₃ antagonist is more preferably, 3-(2-((((1R)-1-(3,5-dimethylphenyl)-3-methylbutyl)amino)carbonyl)-4-(5-fluoro-2-methylphenoxymethyl)phenyl)propanoic acid, 3-(2-(((1R)-3-methyl-1-(3,5-dimethylphenyl)butyl)carbamoyl)-4-(2,5-difluorophenoxymethyl)phenyl)propanoic acid or 3-(2-(((1R)-3-methyl-1-(3,5-dimethylphenyl)butyl)carbamoyl)-4-(2,5-dimethylphenoxymethyl)phenyl)propanoic acid, a salt thereof, a solvate thereof or a prodrug thereof, especially preferably, 3-(2-((((1R)-1-(3,5-dimethylphenyl)-3-methylbutyl)amino)carbonyl)-4-(5-fluoro-2-methylphenoxymethyl)phenyl)propanoic acid, a salt thereof, a solvate thereof or a prodrug thereof.

Unless otherwise specified, the present invention includes all isomers. For example, alkyl includes straight or branched ones. In addition, the present invention also include isomers on double bond, ring, fused ring (*E-, Z-*, cis-, trans-), isomers generated from asymmetric carbon atoms (*R*-, *S-,* α-, β-configuration, enantiomer, diastereomer), optically active isomers (*D-, L-, d-, l*-), polar compounds generated by chromatographic separation (more polar compound, less polar compound), equilibrium compounds, rotational isomers, mixtures thereof at any ratios and racemic mixtures.

In the present invention, unless otherwise specified, as will be apparent to those skilled in the art, a symbol 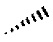 represents bonding to back of the paper (that is, α-configuration), 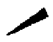 represents bonding to front of the paper (that is, β-configuration), 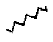 represents α-configuration, β-configuration or a mixture thereof and 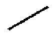 represents mixture of α-configuration and β-configuration. [salt]

The compound of the present invention may be converted into a corresponding pharmaceutically acceptable salt by known methods. Non-toxic and water-soluble salts are preferable. In the present invention, salts include salts of alkali metals, such as potassium, sodium, *etc.*; salts of alkaline-earth metals, such as calcium, magnesium, *etc*.; ammonium salts, pharmaceutically acceptable organic amines, such as tetramethylammonium, triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, *etc.*

In the present invention, preferable acid addition salts are non-toxic and water-soluble salts. In the present invention, acid addition salts include salts of inorganic acids such as hydrochloride, hydrobromide, sulfate, phosphate, nitrate; salts of organic acids, e.g., acetate, lactate, tartrate, oxalate, fumarate, maleate, citrate, benzoate, methanesulphonate, ethanesulphonate, benzenesulphonate, toluenesulphonate, isethionate, glucuronate, gluconate.

The compound of the present invention and a pharmaceutically acceptable salt thereof may be converted into the corresponding solvates.

The solvates are preferably nontoxic and water-soluble. The appropriate solvates include, for example, water, alcohol solvate (such as ethanol) and the like.

Prodrugs of the compounds of the present invention refer to any compounds that are convertible to the compounds represented by the formula (I) and formula (II) through the *in vivo* reactions with enzymes or gastric acid, *etc.* The prodrugs of the compounds of the present invention include, for example, the compounds of the formula (I) and formula (II) having their amino groups acylated, alkylated or phosphated (for example, the compounds represented by the formula (I) having their amino groups eicosanoylated, alanylated, pentylaminocarbonylated, (5-methyl-2-oxo-1,3-dioxolan-4-yl)methoxycarbonylated, tetrahydrofuranylated, pyrrolidylmethylated, pivaloyloxy-methylated, acetoxymethylated, tert-bytylated, and the like); the compounds of the formula (I) and formula (II) having their hydroxyl groups acylated, alkylated, phosphated or borated (for example, the compounds of the formula (I) and formula (II) having their hydroxyl groups acetylated, palmitoylated, propanoylated, pivaloylated, succinylated, furmarylated, alanylated or dimethylaminomethylcarbonylated, and the like); the compounds of the formula (I) and formula (II) having their carboxyl groups esterified or amidated (for example, the compounds of the formula (I) and formula (II) having their carboxyl groups ethyl-esterified, isopropyl-esterified, phenyl-esterified, carboxymethyl-esterified, dimethylaminomethyl-esterified, pivaloyloxymethyl-esterified, ethaxycarbonyloxyethyl-esterified, phthalidyl-esterified, (5-methyl-2-oxo-1,3-dioxolan-4-yl)methyl-esterified or cyclohexyloxycarbonylethyl-esterified, methyl-amidated, and the like); the compounds of the formula (I) and formula (II) having their carboxyl groups replaced with hydroxymethyl group, and the like. These compounds can be produced by the *per se* known methods. Also, the prodrugs of the compounds of the formula (I) and formula (II) may be either hydrates or non-hydrates.

### Method of producing the compound of the present invention:

The compound represented by formula (I) can be produced by the production processes described in WO03/016254 pamphlet and the production processes described in its Examples.

The present compound of formula (II) may be prepared, for example, by the following method.
(1) In the compound of formula (II), wherein R^{1'} is hydrogen, that is, the compound of formula (IIa): 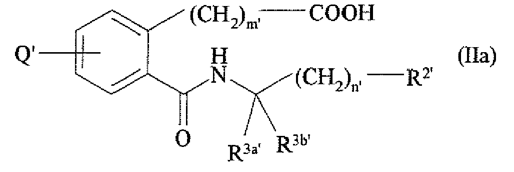
wherein all symbols have the same meanings as described above;
may be prepared by subjecting to deprotection under alkaline conditions the compound of formula (IIb): 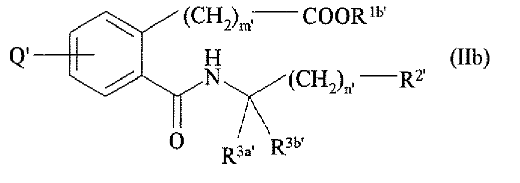 wherein R^{1b'} is C1-4 alkyl and the other symbols have the same meanings as described above.

Deprotection under alkaline conditions is known, for example it may be carried out in water-miscible organic solvent (for example, methanol, ethanol, tetrahydrofuran, dioxane or a mixture thereof), using an aqueous solution of alkali (for example, sodium hydroxide, potassium hydroxide or potassium carbonate) at form -10 to 90°C.

The compound represented by formulae (IIb) may be prepared, for example, by methods in accordance with the following reaction schemes A-D.

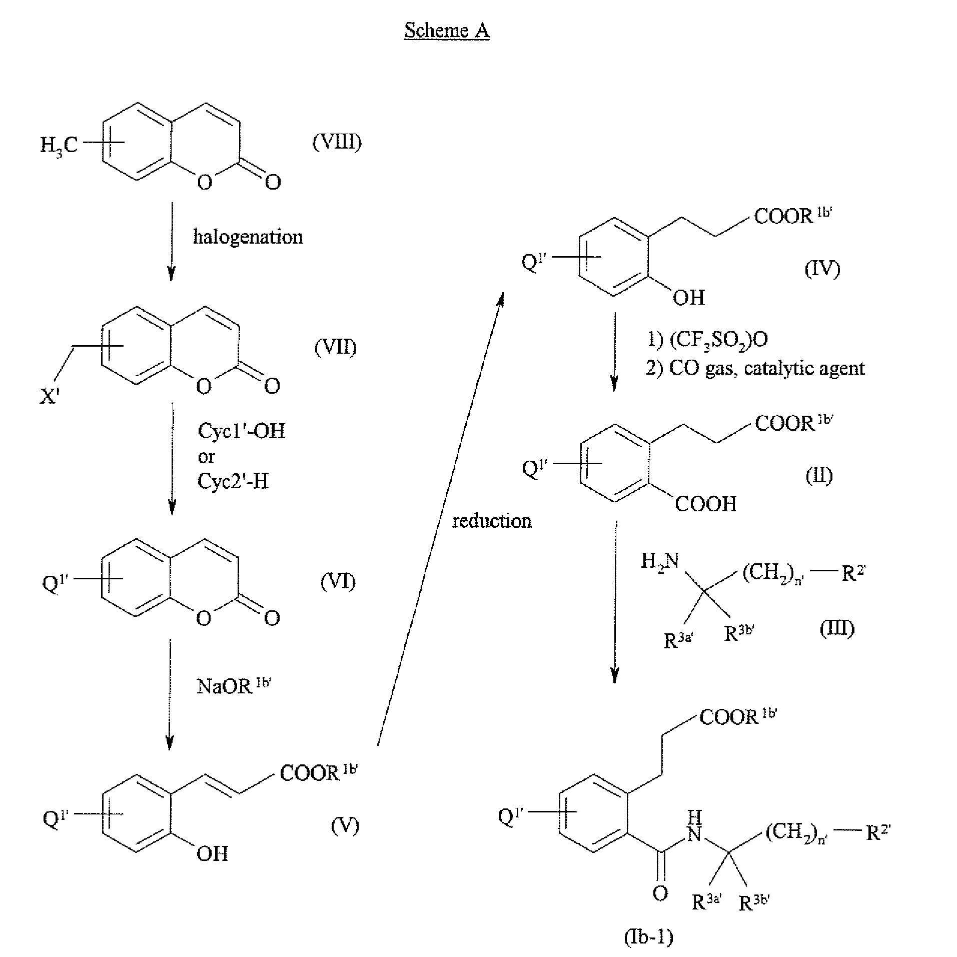

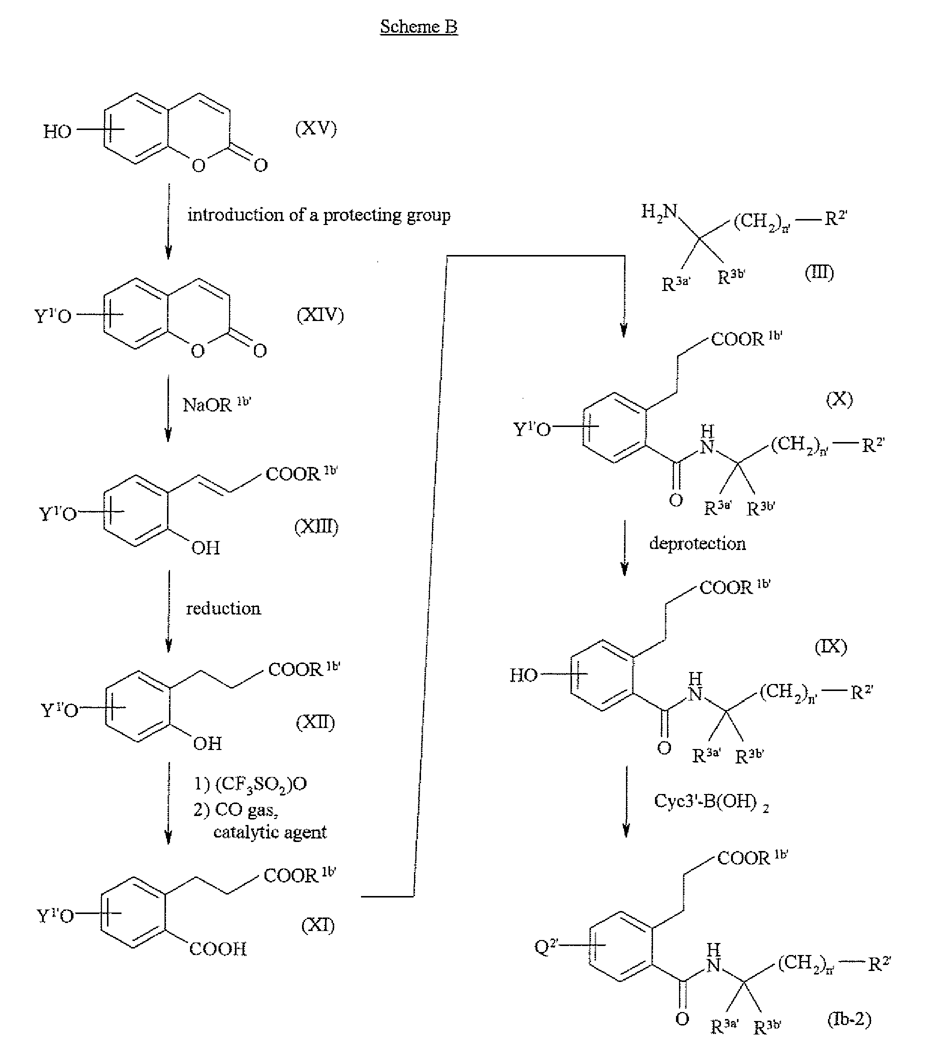

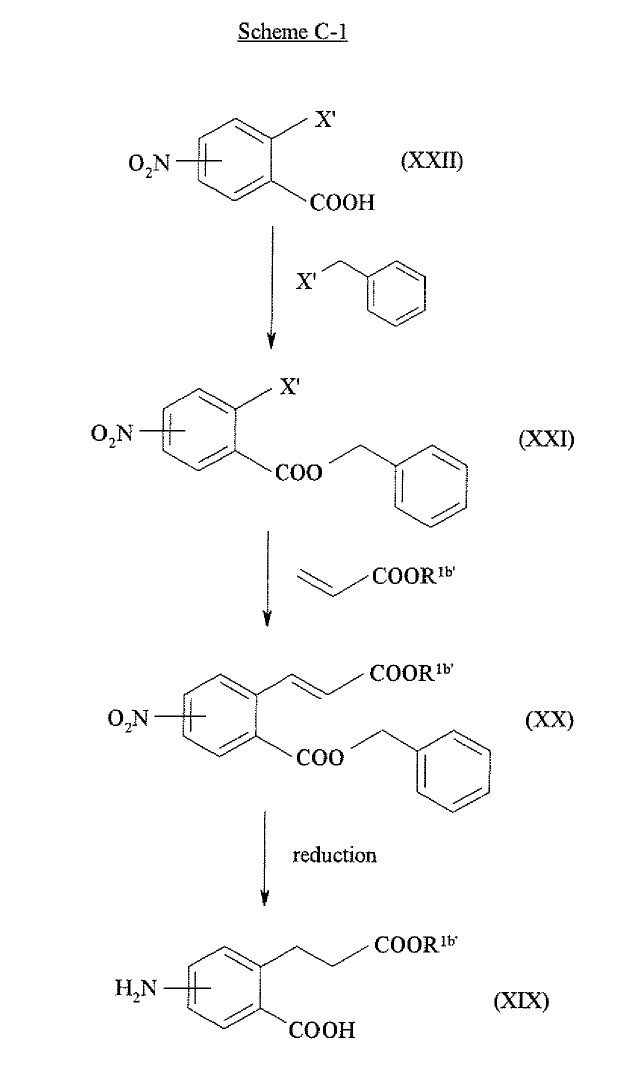

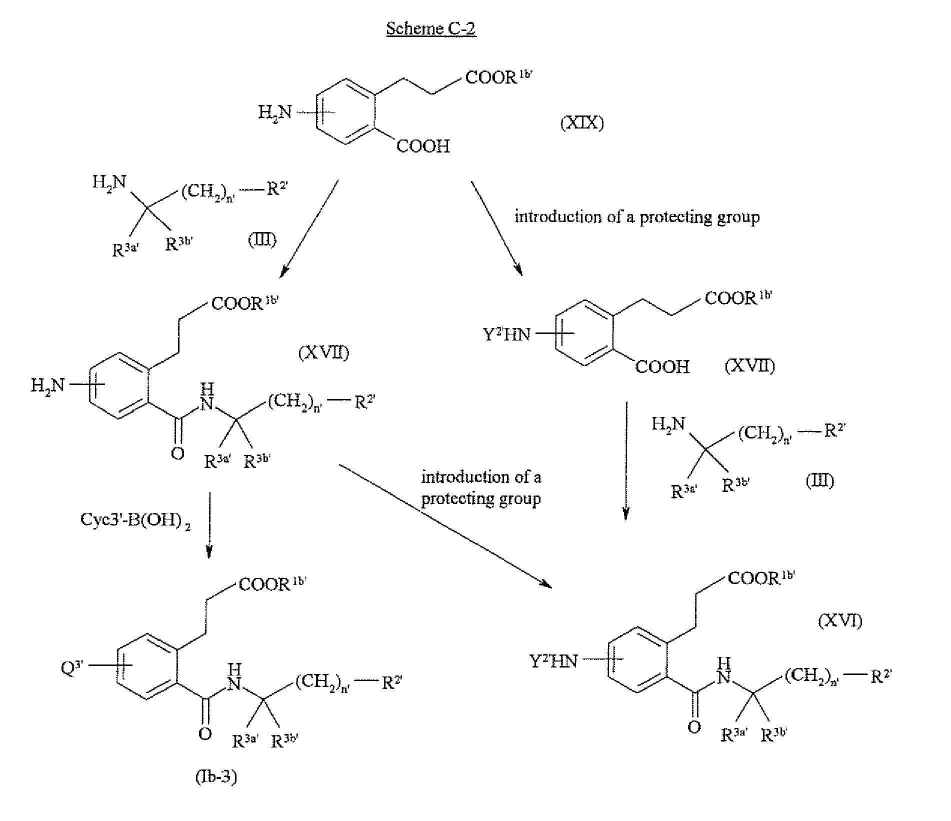

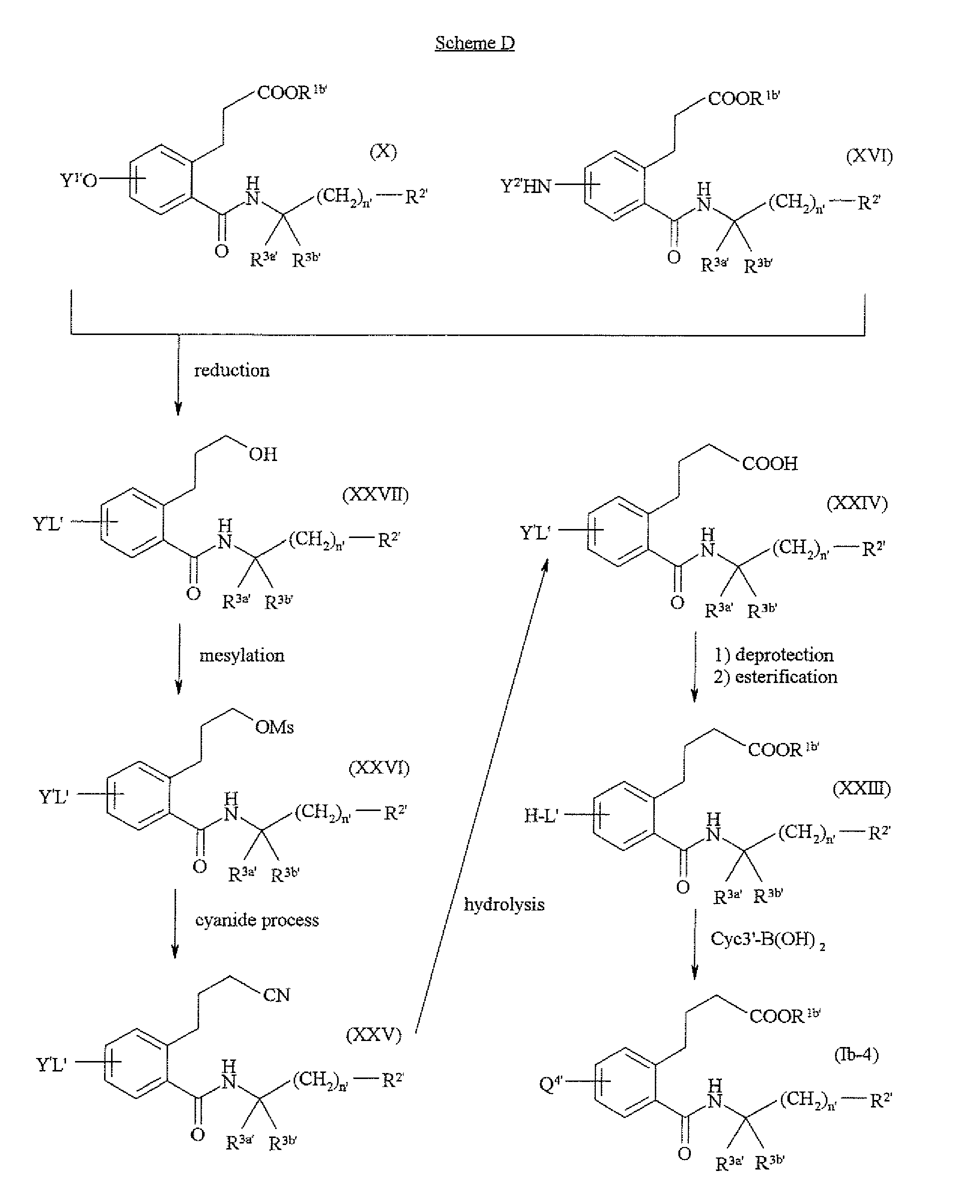

In the schemes,
Q^{1'} is (i) -CH₂-O-Cyc1' or (ii) -CH₂-Cyc2';
Q^{2'} is (iii-1) -O-Cyc3';
Q^{3'} is (iii-2) -NH-Cyc3';
Q^{4'} is (iii) -L'-Cyc3';
Y^{1'} is a protecting group of hydroxyl;
Y^{2'} is a protecting group of amino;
Ms is mesyl;
Tf is trifluoromethylsulfonyl;
X' is halogen;

The other symbols have the same meanings as described above.

In the schemes, the compounds represented by formulae (VIII), (XV), or (XXII) to be used as the starting materials are known compounds, and they can be prepared easily by the combination of known methods.

In the respective reactions described in the present specification, the reaction products can be purified by ordinarily employed purification means, such as distillation under atmospheric pressure or reduced pressure, high-performance liquid chromatography using silica gel or magnesium silicate, thin-layer chromatography, ion exchange resins, scavenger resins or column chromatography or such techniques as washing, recrystallization, *etc*. Purification may be performed in the reaction-by-reaction manner or after completion of several reactions.

In the present specification, other EP₃ antagonist can be produced by the production processes described in WO99/47497 pamphlet, WO00/20371 pamphlet, WO01/62708 pamphlet, WO02/16311 pamphlet and WO02/20462 pamphlet and the production processes described in their Examples. Toxicity:
It has been confirmed that the compounds of the present invention have sufficiently low toxicity and thus are safe enough in using as drugs.

### Application to medicinal drugs:

The compounds of the present invention are considered to be useful as a preventive and/or therapeutic agent for psychoneurotic disorder, psychosomatic disorder, anxiety disorder, depression and disorder caused by stress.

In the present specification, the disorder caused by stress refers to the disorder caused by physical and/or mental stress, includes central and/or systemic disorder.

In the present specification, the depression includes a depressive episode, a dysthymic disorder, a cyclothymic disorder, a bipolar emotional disorder or a manic-depression disorder.

The depression includes the case of one or more selected from psychomotor inhibition, anxiety, impatience, idea of belittlement, suicidal ideation, headache, insomnia, generalized fatigability, feeling of fatigue, febricula, vertigo, tinnitus, stiff shoulder, dry mouth feeling, taste abnormality, palpitatio cordis, dyspnoea, low back pain, arthralgia, reproductive hypesthesia, decreased libido, menstrual disorder, dysfunctional voiding, asitia, nausea, vomiting, abdominal pain, ulcus, abdominal discomfort and coldness of limbs, *etc.*

In the present specification, the anxiety disorder include the case of one or more selected from obsessive-compulsive disorder, panic disorder, posttraumatic stress disorder, generalized anxiety disorder, mixed anxiety depressive disorder, social avoidance and distress and anthrophobia, *etc*.

In the present specification, the psychosomatic disorder include the case of one or more selected from autonomic dystonia, climacteric disorder, hypertension, hypotension, angina, asthma, hyperventilation syndrome, irritable bowel syndrome, gastric ulcer, duodenal ulcer, headache, migraine, over active bladder, enuresis, insomnia, alopecia areata, diabetes, premenstrual syndrome, dysmenorrheal, infertility, alexithymia, alcohol dependence syndrome, anorexia nervosa, bulimia nervosa, Meniere's syndrome, cervicobrachial syndrome and indefinite complaint, *etc*.

Psychosomatic disorder, anxiety disorder or depression described above includes the case of sideration caused by stress.

The compound of the present invention, a salt thereof, a solvate thereof or a prodrug thereof may be administered as a combination preparation by combining with other pharmaceuticals for the purpose of
1) supplementating and/or enhancing the preventive and/or treatment effect of the therapeutic agent represented by the present specification
2) improving pharmacokinetics and absorption of the compound, and reducing the dose of the therapeutic agent represented by the present specification, and/or
3) reducing side effect of the therapeutic agent represented by the present specification.

The combination preparations of the compound of the present invention, a salt thereof, a solvate thereof or a prodrug thereof and a concomitant drug(s) may be administered as one combination preparation comprising these components, or may be administered separately. When they are administered separately as independent preparations, they may be administered simultaneously or with time lag. Administration with time lag includes the method of administering the therapeutic agent represented by the present specification before other drugs and vice versa, and each administration route may be the same or different. The concomitant drug(s) described above may be a low molecular weight compound, and may be a macromolecule protein, polypeptide, polynucleotide (DNA, RNA, gene), antisense, decoy, antibody, vaccine and the like. The dosage of the concomitant drug(s) can be properly selected according to the clinical dosage. The compounding ratio of the therapeutic agent represented by the present specification and the concomitant drug(s) can be properly selected by the age and body weight of the object, administration route, administration term, target disease, symptom, combination and the like. For example, the concomitant drug(s) may be used in an amount of 0.01 parts by weight to 100 parts by weight relative to 1 part by weight of the therapeutic agent represented by the present specification. The concomitant drug(s) may be administrated in the proper combination of arbitrary two or more member(s) selected from an arbitrary proportion. Furthermore, the concomitant drug(s) for supplementation and/or enhancement of the prophylactic and/or therapeutic effect of the therapeutic agent represented by the present specification includes not only those which have so far been found but also those which will be found on the basis of the aforementioned mechanism.

There is no limitation on a disease on which the combination preparations of the therapeutic agent represented by the present specification and a concomitant drug(s) have preventive and/or treatment effects, so long as the preventive and/or treatment effect of the combination preparation is supplemented and/or enhanced in the disease.

Examples of the concomitant drug(s) for supplementing and/or enhancing the preventive and/or therapeutic effect of the compound of the present invention on psychoneurotic diseases, antidepressant agent, for example, tricyclic antidepressant, tetracyclic antidepressant, monoamine oxidaze (MAO) inhibitor, serotonin and noradrenaline reuptake inhibitor (SNRI), selective serotonin reuptake inhibitor (SSRI), S-HT_{1A} receptor agonists, GABA receptor agonists, dopamine receptor antagonist, psychoanaleptic, antianxiety agent, antipsychotic agent, mitochondrial benzodiazepine receptor (MBR) ligand, NK1 antagonist, σ receptor agonists, serotonin nervous system agonists, corticotropin releasing factor (CRF) receptor antagonists, proton pump inhibitors, histamine H₂-receptor antagonist, M1 receptor antagonists, EP₁ antagonist and other therapeutic agent for stress-related disorder.

Examples of the antidepressant agent include tricyclic antidepressant, for example, amitriptyline hydrochloride, imipramine hydrochloride, clomipramine hydrochloride, dosulepin hydrochloride, nortriptyline hydrochloride, lofepramine hydrochloride, trimipramine maleate, amoxapine, anafranil, tetracyclic antidepressant, for example, maprotiline hydrochloride, mianserin hydrochloride, setiptiline maleate, MAO inhibitor, safrazine hydrochloride, selegiline hydrochloride, moclobemide, toloxatone, SNRI, for example, milnacipran hydrochloride, venlafaxine hydrochloride, atomoxetine hydrochloride, bupropion, reboxetine, citalopram hydrobromide, SSRI, for example, fluvoxamine maleate, paroxetine hydrochloride, fluoxetine hydrochloride, citalopram hydrochloride, minaprineand hydrochloride, sibutramine hydrochloride, tianeptine, nefazodone hydrochloridethe, sertraline hydrochloride, trazodone hydrochloride, 5-HT_{1A} receptor agonists, for example, mirtazapine, GABA receptor agonists, for example, progabide, gabapentine, topiramate, dopamine receptor antagonist, for example, risperidone, olanzapine, quetiapine fumarate, ziprasidone hydrochloride hydrate, pramipexole hydrochloride hydrate, talipexole hydrochloride, aripiprazole, levosulpiride, and the like.

Examples of the psychoanaleptic include methylphenidate hydrochloride, pemoline, and the like.

Examples of the antianxiety agent include benzothiazepine, for example, alprazolam, oxazepam, oxazolam, cloxazolam, clorazepate dipotassium, chlordiazepoxide, diazepam, tofisopam, triazolam, prazepam, fludiazepam, flutazolam, flutoprazepam, bromazepam, mexazolam, medazepam, ethyl loflazepate, lorazepam, clonazepam, adinazolam mesilate, thienodiazepine class, for example, etizolam, clotiazepam, nonbenzodiazepine, for example, tandospirone citrate, hydroxyzine hydrochloride, buspirone, venlafaxine, tropisetron hydrochloride, alosetron, tiagabine hydrochloride, and the like.

Examples of the antipsychotic agent include sulpiride, trazodone hydrochloride, serotonin/dopamine antagonist, for example, risperidone, perospirone hydrochloride hydrate, quetiapine fumarate, olanzapine, and the like.

Examples of the gastrointestinal promotility agent include trimebutine maleate, polycarbophil calcium, and the like.

Examples of the 5-HT₃ receptor antagonists include alosetron, and the like.

Examples of the 5-HT₄ receptor agonists include tegaserod, cisapride, mosapride citrate, and the like.

Examples of other therapeutic agent for stress-related disorder include tandospirone citrate, lesopitron, igmesine, AP-521, PLD-116, ilaprazole, ME-3412, DMP-696, ME-3412, YJA-20379-8, pirenzepine hydrochloride, lansoprazole, dosmalfate and osemozotan.

Examples of the therapeutic agent for bipolar emotional disorder include sultopride hydrochloride, carbamazepine, valproate sodium, and the like.

The compound of the present invention, a salt thereof, a solvate thereof, or a prodrug thereof can be useful as a preventive and/or therapeutic agent for psychoneurotic disorder, psychosomatic disorder, anxiety disorder, depression and disorder caused by stress, because of safety and low toxicity. Presently, the antidepressant agent and the antianxiety agent used as a therapeutic agent for psychoneurotic diseases are known that sleepiness, sensation of thirst, and the like may occur as a side effect. However, the compound of the present invention has an antagonistic potency against EP₃ and no side effect induced by the existing agent, because of working mechanism different from these existing agents.

When the compound of the present invention, a salt thereof, a solvate thereof, or a prodrug thereof is used as an active ingredient, and it may be administered alone or mixture of various types of pharmacological acceptable pharmaceutic aid as an pharmaceutical composition. Generically, it is administered to a mammal, for example, human, nonhuman animal, and the like, and preferably, human, as oral administration, intracavernous administration, local administration, dermal administration, and it can be used as the usable type of the preparation of the drug product for any purpose

The dose of these compounds to be administered are determined depending upon, for example, age, body weight, symptom, the desired therapeutic effect, the route of administration, the duration of the treatment, and the like. For a human adult, generally about 1 µg to about 1 g per dose is orally administered once several days, once thrice days, once twice days, once to several times a day, or about 1 µg to about 300 mg per dose is parenterally (preferably intravenously) administered once several days, once thrice days, once twice days, once to several times a day, or intravenously administered continuously for 1 to 24 hours a day.

It goes without saying that the dose of these compounds may be less than the aforementioned value or may need to exceed the aforementioned range because the dose varies under various conditions, condition of the disease and age, as mentioned above

In contrast, the dosage in human can be estimated or determined scientific based on the dose in nonhuman animal, for example, with the dose in rat model, and it is based on factual evidence or empirical foundation. For example, by an experiment with the use of an olfactory bulbectomy rat in the present invention, desipramine hydrochloride salt was effective in 20mg/kg per day. However, the adult dosage of desipramine hydrochloride salt in human is 100 mg to 200 mg per day [*Physicians' Desk Reference,* 57Edition, 2003]. Therefore, the daily dosage in human is 5 to 10 times the dosage excepted weight ratio in the rat model (that is to say, 20 mg). If these scientific bases are applied to the compound of the present invention, it can be expected that the daily dosage in human is 1 to 5 to 10 times the dosage excepted weight ratio in the rat model, because of the effectiveness of the daily dosage of 10 mg/kg in the rat model. That is to say, it can be determined that a preferable dosage in human is about 10 mg to about 100 mg per day.

If this scientific prediction is applied to the present invention, the dosage of the compound of the present invention as a preventive and/or therapeutic agent for psychoneurotic disorder is preferably the total amount of about 1 mg to about 600 mg in a day, and more preferably, about 5 mg to about 300 mg, especially preferably, about 10 mg to about 100 mg, most preferably, about 10 mg to about 50 mg by administering orally daily in once to quarter divided doses. Furthermore, a solid preparation includes, preferably the amount of about 1 mg to about 100 mg of the compound of the present invention. And more preferably, it includes about 2.5 mg per solid preparation, about 5 mg per solid preparation, about 10 mg per solid preparation, about 25 mg per solid preparation and about 50 mg per solid preparation. These solid preparations may be administered one or a combination of more than the arbitrary ones. The solid preparation is preferably tablets, capsules, and especially preferably, tablets, and it is preferred that the solid preparation is administered daily in one to three times divided doses, each one to four tablets. And the solid preparation may be administered one to twelve tablets per day.

In the administration of the combination preparation of the compound of the present invention, a salt thereof, a solvate thereof, or a prodrug thereof and the concomitant drug(s) can be administered as solid agents for oral administration, liquid preparations for internal use and injections for parenteral administration, external preparation, suppositories, eye-drops, respiratory tonic, and the like.

Solid agents for oral administration include tablets, pills, capsules, dispersible powders and granules. Capsules include hard capsules and soft capsules, while the tablets include sublingual tablets, tablets for intraoral strapping and intraorally rapid- disintegrating tablets.

In such solid agents, one or more of the active compound(s) may be alone, or admixed with vehicles (such as lactose, mannitol, glucose, microcrystalline cellulose, starch, *etc*.), binders (such as hydroxypropyl cellulose, polyvinylpyrrolidone, magnesium metasilicate aluminate, *etc*.), disintegrants (such as cellulose calcium glycolate, *etc*.), lubricants (such as magnesium stearate, *etc*.), stabilizers, solubilizing agents (such as glutamic acid, aspartic acid, *etc.)* and formulated according to common methods. The solid agents may, if desired, be coated with coating agents (such as white sugar, gelatin, hydroxypropyl cellulose, hydroxypropylmethyl cellulose phthalate, *etc.),* or be coated with two or more films. And further, coating may include containment within capsules of absorbable materials such as gelatin.

Sublingual tablets can be carried out by the known method. For example, one or more of the active compound(s) may be admixed with vehicles (such as lactose, mannitol, glucose, microcrystalline cellulose, colloidal silica, starch, *etc*.), binders (such as hydroxypropyl cellulose, polyvinylpyrrolidone, magnesium metasilicate aluminate, *etc.*), disintegrants (such as starch, L-hydroxypropyl cellulose, carboxymethyl cellulose, croscarmellose sodium, cellulose calcium glycolate, *etc.*), lubricants (such as magnesium stearate, *etc.*), swelling agents (such as hydroxypropyl cellulose, hydroxypropylmethyl cellulose, carbopole, carboxymethyl cellulose, polyvinyl alcohol, xanthan gum, guar gum, *etc.*), swelling assistance agents (such as glucose, fructose, mannitol, xylitol, erythritol, maltose, trehalose, phosphate, citrate, silicate, glycine, glutamic acid, arginine, *etc*.) stabilizers, solubilizing agents (such as polyethylene glycol, propylene glycol, glutamic acid, aspartic acid, *etc*.), flavoring (such as orange, strawberry, mint, lemon, vanilla, *etc.)* and formulated according to common methods. The solid agents may, if desired, be coated with coating agents (such as white sugar, gelatin, hydroxypropyl cellulose, hydroxypropylmethyl cellulose phthalate, *etc.*), or be coated with two or more films. Furthermore, the solid agents may, if necessary, include habitual preservatives, antioxidants, coloring agents, sweetening agents, and the like.

Tablets for intraoral strapping can be carried out by the known method. For example, one or more of the active compound(s) may be admixed with vehicles (such as lactose, mannitol, glucose, microcrystalline cellulose, colloidal silica, starch, *etc.*), binders (such as hydroxypropyl cellulose, polyvinylpyrrolidone, magnesium metasilicate aluminate, *etc.*), disintegrants (such as starch, L-hydroxypropyl cellulose, carboxymethyl cellulose, croscarmellose sodium, cellulose calcium glycolate, *etc.*), lubricants (such as magnesium stearate, *etc.*), attaching agent (such as hydroxypropyl cellulose, hydroxypropylmethyl cellulose, carbopole, carboxymethyl cellulose, polyvinyl alcohol, xanthan gum, guar gum, *etc*.), attaching assistance agents (such as glucose, fructose, mannitol, xylitol, erythritol, maltose, trehalose, phosphate, citrate, silicate, glycine, glutamic acid, arginine, *etc.*), stabilizers, solubilizing agents (such as polyethylene glycol, propylene glycol, glutamic acid, aspartic acid, *etc.*), flavoring (such as orange, strawberry, mint, lemon, vanilla, *etc*.) and formulated according to common methods. The solid agents may, if desired, be coated with coating agents (such as white sugar, gelatin, hydroxypropyl cellulose, hydroxypropylmethyl cellulose phthalate, *etc.),* or be coated with two or more films. Furthermore, the solid agents may, if necessary, include habitual preservatives, antioxidants, coloring agents, sweetening agents, and the like.

Intraorally rapid-disintegrating tablets can be carried out by the known method. For example, one or more of the active compound(s) may be alone, or the active compound which the bulk powder or the granulated bulk powder particle are coated with the suitable coating agents (such as ethyl cellulose, hydroxypropyl cellulose, hydroxypropylmethyl cellulose, acrylic acid methacrylate copolymer, *etc*.), plasticizing agent (such as polyethylene glycol, triethyl citrate, *etc.)* may be admixed with vehicles (such as lactose, mannitol, glucose, microcrystalline cellulose, colloidal silica, starch, *etc.),* binders (such as hydroxypropyl cellulose, polyvinylpyrrolidone, magnesium metasilicate aluminate, *etc.*), disintegrants (such as starch, L-hydroxypropyl cellulose, carboxymethyl cellulose, croscarmellose sodium, cellulose calcium glycolate, *etc.*), lubricants (such as magnesium stearate, *etc.*), disintegrating assistance agents (such as glucose, fructose, mannitol, xylitol, erythritol, maltose, trehalose, phosphate, citrate, silicate, glycine, glutamic acid, arginine, *etc.*), stabilizers, solubilizing agents (such as polyethylene glycol, propylene glycol, glutamic acid, aspartic acid, *etc.),* flavoring (such as orange, strawberry, mint, lemon, vanilla, *etc.*) and formulated according to common methods. The solid agents may, if desired, be coated with coating agents (such as white sugar, gelatin, hydroxypropyl cellulose or hydroxypropylmethyl cellulose phthalate, *etc.),* or be coated with two or more films. Furthermore, the solid agents may, if necessary, include habitual preservatives, antioxidants, coloring agents, sweetening agents, and the like.

Liquid agents for oral administration include pharmaceutically acceptable solutions, suspensions, emulsions, syrups, elixirs, *etc.* In such liquid agents, one or more of the active compound(s) may be dissolved, suspended or emulsified into diluent(s) commonly used in the art (such as purified water, ethanol or a mixture thereof). The liquid agents may further comprise some additives, such as wetting agents, suspending agents, emulsifying agents, sweetening agents, flavoring agents, aroma, preservatives or buffering agents.

The dosage forms of the parenteral administration preparations for external use include ointments, gels, creams, fomentations, patches, liniments, atomized agents, inhalations, sprays, aerosols, eye-drops, nasal drops and the like. Such a preparation contains one or two or more active substances and is prepared by a well known method or a commonly employed formulation.

Ointments are prepared in accordance with a well known formulation or a commonly employed formulation. For example, they are prepared by softening or melting one or two or more active substances in a base. The ointment base is selected from well known ones or those commonly employed. For example, use may be made of one base or a mixture of two or more thereof selected from higher fatty acids or higher fatty acid esters (adipic acid, myristic acid, palmitic acid, stearic acid, oleic acid, adipic acid esters, myristic acid esters, palmitic acid esters, stearic acid esters, oleic acid esters, *etc*.), waxes (beeswax, whale wax, ceresin, *etc*.), surfactants (polyoxyethylene alkyl ether phosphoric acid esters, *etc*.), higher alcohols (cetanol, stearyl alcohol, cetostearyl alcohol, *etc*.), silicone oils (dimethylpolysiloxane, *etc*.), hydrocarbons (hydrophilic vaseline, white vaseline, refined lanolin, liquid paraffin, *etc*.), glycols (ethylene glycol, diethylene glycol, propylene glycol, polyethylene glycol, macrogol, *etc*.), vegetable oils (castor oil, olive oil, sesame oil, turpentine oil, *etc.*), animal oils (mink oil, yolk oil, squalane, squalene, *etc*.), water, absorption promoters and skin irritation inhibitors. The ointments may further contain a humectant, a preservative, a stabilizer, an antioxidant, a flavor, and the like.

Gels are prepared in accordance with a well known formulation or a formulation commonly employed. For example, they are prepared by melting one or more active substances in a base. The gel base is selected from well known ones or those commonly employed. For example, use may be made of one base or a mixture of two or more thereof selected from lower alcohols (ethanol, isopropyl alcohol, *etc.),* gelling agents (carboxymethylcellulose, hydroxyethylcellulose, hydroxypropylcellulose, ethylcellulose, *etc*.), neutralizing agents (triethanolamine, diisopropanolamine, *etc*.), surfactants (polyethylene glycol monostearate, *etc*.), gums, water, absorption promoters and skin irritation inhibitors. The gels may further contain a preservative, an antioxidant, a flavor, and the like.

Creams are prepared in accordance with a well known formulation or a formulation commonly employed. For example, they are prepared by melting or emulsifying one or more active substances in a base. The cream base is selected from well known ones or those commonly employed. For example, use may be made of one base or a mixture of two or more thereof selected from higher fatty acid esters, lower alcohols, hydrocarbons, polyhydric alcohols (propylene glycol, 1,3-butylene glycol, *etc.*), higher alcohols (2-hexyldecanol, cetanol, *etc.*), emulsifiers (polyoxyethylene alkyl ethers, fatty acid esters, *etc.*), water, absorption promoters and skin irritation inhibitors. The creams may further contain a preservative, an antioxidant, a flavor, and the like.

Fomentations are prepared in accordance with a well known formulation or a formulation commonly employed. For example, they are prepared by melting one or more active substances in a base, kneading and then applying and spreading the kneaded matter on a substrate. The fomentation base is selected from well known ones or those commonly employed. For example, use may be made of one base or a mixture of two or more thereof selected from thickeners (polyacrylic acid, polyvinylpyrrolidone, gum acacia, starch, gelatin, methylcellulose, *etc.*), moistening agents (urea, glycerin, propylene glycol, *etc.),* fillers (kaolin, zinc oxide, talc, calcium, magnesium, *etc*.), water, dissolution aids, tackifiers and skin irritation inhibitors. The fomentations may further contain a preservative, an antioxidant, a flavor, and the like.

Patches are prepared in accordance with a well known formulation or a formulation commonly employed. For example, they are prepared by melting one or more active substances in a base and then applying and spreading on a substrate. The patch base is selected from well known ones or those commonly employed. For example, use may be made of one base or a mixture of two or more thereof selected from polymer bases, fats and oils, higher fatty acids, tackifiers and skin irritation inhibitors. The patches may further contain a preservative, an antioxidant, a flavor, and the like.

Liniments are prepared in accordance with a well known formulation or a formulation commonly employed. For example, they are prepared by dissolving, suspending or emulsifying one or two or more active substances in one or more media selected from water, alcohols (ethanol, polyethylene glycol, *etc.*), higher fatty acids, glycerin, soap, emulsifiers, suspending agents, and the like. The liniments may further contain a preservative, an antioxidant, a flavor, and the like.

Atomized agents, inhalations and sprays may contain, in addition to a diluent commonly employed, a stabilizer such as sodium hydrogen sulfite, a buffering agent for imparting isotonicity, for example, an isotonic agent such as sodium chloride, sodium citrate or citric acid. Methods for producing a spray are described in detail in, for example, U.S. Patent No. 2,868,691 and U.S. Patent No. 3,095,355.

Injections for parenteral administration include any types of injections including drops. Examples of injections include intramuscular injections, subcutaneous injections, intradermal injections, intraarterial injections, intravenous injections, intraabdominal injections, intraspinal injections, intravenous drips, *etc.*

The injections for parenteral administration include solutions, suspensions, emulsions and solid injections to be dissolved or suspended before use. Such an injection is used by dissolving, suspending or emulsifying one or more active substances in a solvent. The solvent includes, for example, distilled water for injection, physiological saline, vegetable oils, alcohols such as propylene glycol, polyethylene glycol and ethanol, and mixtures thereof. The injection may further contain a stabilizer, a dissolution aid (glutamic acid, aspartic acid, Polysorbate 80 (registered trademark), *etc.*), a suspending agent, an emulsifier, a soothing agent, a buffer, a preservative, and the like. Such an injection may be produced by sterilizing at the final step or employing an aseptic process. Alternatively, it is also possible that an aseptic solid product such as a freeze-dried product is produced and sterilized or dissolved in aseptic distilled water for injection or another solvent before use.

The eye-drops for parenteral administration include ophthalmic solution, suspending ophthalmic solution, emulsifying ophthalmic solution, ophthalmic solution to be dissolved before use or ophthalmic ointment.

The eye-drops may be prepared in accordance with a well known method. For example, the eye-drops are used by dissolving, suspending or emulsifying one or more active substances in a solvent. The solvent of eye-drops includes, for example, sterile purified water, physiological saline, other water-based solvents or no aqueous solvents for the injection (for example, vegetable oils, *etc.),* and the like, and mixtures thereof. The eye-drops may accordingly contain tonicity agents (for example, sodium chloride, concentrated glycerin, *etc*.), buffering agents (for example, sodium phosphate, sodium acetate, *etc*.), surface acting agents (for example, Polysorbate 80 (registered trademark), polyoxyl stearate 40, polyoxyethylene hardening of castor oil, *etc.),* stabilizers (for example, sodium citrate, edetate sodium, *etc.*), antiseptic agents (for example, benzalkonium chloride, paraben, *etc.),* and the like as needed. The eye-drops may be produced by sterilizing at the final step or employing an aseptic process. Alternatively, it is also possible that an aseptic solid product such as a freeze-dried product is produced and sterilized or dissolved in aseptic sterile purified water or another solvent before use.

The inhalations for parenteral administration include aerosols, powders for inhalation and liquids for inhalation. Such inhalations may be dissolved or suspended in water or another adequate medium for use.

The inhalations may be prepared in accordance with a well known method.

For example, liquid preparations for inhalation may be, if necessary, prepared by appropriately selecting a preservative (benzalkonium chloride, paraben, *etc.),* a colorant, a buffering agent (sodium phosphate, sodium acetate, *etc*.), an isotonic agent (sodium chloride, concentrated glycerin, *etc.*), a thickener (carboxyvinyl polymer, *etc.),* an absorption promoter, and the like.

Powders for inhalation may be prepared, if necessary, by appropriately selecting a lubricant (stearic acid and its salt, *etc.),* a binder (starch, dextrin, *etc.*), an excipient (lactose, cellulose, *etc.*), a colorant, a preservative (benzalkonium chloride, paraben, *etc.*), an absorption promoter, and the like.

When the liquids for inhalation are administered, a sprayer (atomizer, nebulizer) is usually used. When the powders for inhalation are used, an inhalation administration apparatus for powder agents is usually used.

Other compositions for parenteral administration include suppositories and pessaries for vaginal administration, which contain one or more active substances, and are prepared in accordance with common formulations.

### EFFECT OF THE INVENTION

The compound of the present invention is useful as a preventive and/or therapeutic agent for psychoneurotic disorder, psychosomatic disorder, anxiety disorder, depression and disorder caused by stress without side effect.

### BRIEF DESCRIPTION OF THE DRAWINGS

Figure 1 shows the result that corticosterone elevation was inhibited by the administration of 10 mg/kg of 3-(2-((((1R)-1-(3,5-dimethylphenyl)-3-methylbutyl)amino)carbonyl)-4-(5-fluoro-2-methylphenoxymethyl)phenyl)propanoic acid (compound (I)), 3-(2-(((1R)-3-methyl-1-(3,5-dimethylphenyl)butyl)carbaznoyl)-4-(2,5-difluorophenoxymethyl)phenyl)propanoic acid (compound (II)) and 3-(2-(((1R)-3-methyl-1-(3,5-dimethylphenyl)butyl)carbamoyl)-4-(2,5-dimethylphenoxymethyl)phenyl)propanoic acid (compound (III)).
Figure 2 shows the result that score of emotional responses was decreased significantly by administration of the compound (I).
Figure 3 shows the result that the reduction of the time in the open arms on the elevated plus-maze was improved significantly by an administration of the compound (I).

### BEST MODE FOR CARRYING OUT THE INVENTION

The following Examples and Formulation Example are provided to illustrate the present invention in detail, but are not to be construed as limiting the invention.

The name of the compounds used in the present specification is designated according to IUPAC regulations.

Solvents given in parentheses concerning chromatographic separation and TLC indicate each the elution solvent or the developing solvent employed and the ratio is expressed in ratio by volume.

Solvents given in parentheses concerning NMR indicate each the solvent employed in measurement.

### Example 1

### 7-bromomethylcoumarin:

To a solution of 7-methylcoumarin (50 g) in acetonitrile (1.2 L), N-bromosuccinimide (56 g) and α,α'-azobisisobutylonitrile (510 mg) were added, and the mixture was stirred for 30 minutes at 78°C of inside temperature. The reaction mixture was concentrated, and water was added. The crystals were collected by filtration to give the title compound (76 g) having the following physical data. NMR (300 MHz, CDCl₃): δ 7.69, 7.46, 7.34, 7.30, 6.43, 4.52.

### Example 2

### 7-(2, 5-difluorophenoxymethyl)coumarin:

The compound prepared in Example 1 (40 g), 2,5-difluorophenol (21.8 g) and potassium carbonate (46.4 g) were dissolved in dimethylformamide (DMF; 250 mL), and the mixture was heated for 50 minutes at 60°C. After the reaction mixture was cooled at room temperature, water was added to the mixture. Solid was collected by filtration. The solid was dried to give the title compound (43.9 g) having the following physical data.
NMR (300 MHz, DMSO-d₆): δ 8.05, 7.74, 7.46, 7.41, 7.32-7.18, 6.78, 6.49, 5.30.

### Example 3

### 3-(4-(2,5-difluorophenoxymethyl)-2-hydroxyphenyl)propenoic acid methyl ester:

Under an atmosphere of argon, to a solution of sodium hydride (18.2 g, 60% oil) in tetrahydrofuran (THF; 150 mL), methanol (24.6 mL) was added at room temperature. The mixture was stirred for 30 minutes at 50°C. The mixture was cooled to room temperature, and a solution of the compound prepared in Example 2 (43.9 g) in DMF (750 mL) was added dropwise to the mixture. The mixture was stirred for 30 minutes at 50°C. The mixture was cooled to room temperature. 1N Hydrochloric acid was added to the mixture in ice-bath. The mixture was extracted with ethyl acetate. The organic layer was washed with water and a saturated aqueous solution of sodium chloride, dried over anhydrous magnesium sulfate and concentrated. An obtained solid was crystallized by a mixture of t-butyl methyl ether / hexane to give the title compound (46.5 g) having the following physical data.
NMR(300 MHz, DMSO-d₆): δ 10.4, 7.84, 7.64, 7.26, 7.16, 6.98, 6.89, 6.77, 6.61, 5.15, 3.70.

### Example 4

### 3-(4-(2,5-difluorophenoxymethyl)-2-hydroxyphenyl)propanoic acid methyl ester:

To a solution of the compound prepared in Example 3 (46.5 g) in THF (400 mL)/methanol (100 mL), dichloro nickel six hydrous (41.3 g) and sodium borohydride (21.9 g) were slowly added. The mixture was stirred for 2.5 hours. The reaction solution was diluted with t-butyl methyl ether and filtered through celite (trade mark). The filtrate was diluted with ethyl acetate, washed with water and a saturated aqueous solution of sodium chloride, dried over magnesium sulfate and concentrated. The residue was purified by column chromatography on silica gel (ethyl acetate: hexane = 1: 1) to give the title compound (23.6 g) having the following physical data. NMR (300 MHz, CDCl₃): δ 7.20, 7.10, 7.01, 6.96-6.91, 6.71, 6.58, 5.03, 3.70, 2.92-2.88, 2.74-2.70.

### Example 5

### 3-(2-carboxy-4-(2,5-difluorophenoxymethyl)phenyl)propanoic acid methyl ester:

Under an atmosphere of argon, to a solution of the compound prepared in Example 4 (250 mg) in pyridine (1.55 mL), trifluoromethanesulfonate (144 µL) was added at 0°C. The mixture was stirred for 60 minutes at room temperature. The reaction mixture was diluted with ethyl acetate. The dilute solution was washed with water, 1N hydrochloric acid, water and a saturated aqueous solution of sodium chloride, successively, dried over magnesium sulfate and concentrated.

Under an atmosphere of argon, to a solution of the obtained compound in DMF (2.5 mL), potassium acetate (380 mg), bis(diphenylphosphino)ferrocene (41 mg) and palladium acetate (II) (8.7 mg) were added successively. The mixture was stirred overnight at 90°C under an atmosphere of carbon monoxide gas. The reaction mixture was diluted with t-butyl methyl ether, and filtered through celite (trade mark). The filtrate was washed with a saturated ammonium chloride solution, water and a saturated aqueous solution of sodium chloride, successively, dried over magnesium sulfate and concentrated. The residue was purified by column chromatography on silica gel (ethyl acetate: hexane = 1: 1) to give the title compound (193 mg) having the following physical data.
NMR (300 MHz, CDCl₃): δ 8.11, 7.59, 7.38, 7.04, 6.74, 6.62, 5.11, 3.67, 3.38-3.33, 2.74-2.69.

### Example 6

### 3-(4-(2,5-difluorophenoxymethyl)-2-((((1R)-1-(naphthalen-2-yl)ethyl)amino)carbonyl)phenyl)propanoic acid methyl ester:

Under an atmosphere of argon, a solution of the compound prepared in Example 5 (80 mg), (1R)-1-(naphthalen-2-yl)ethylamine (47 mg), 1-ethyl-3-[3-(dimethylamino)propyl]carbodiimide (66 mg) and 1-hydroxybenzotriazole (46 mg) in DMF (1 mL) was stirred overnight at room temperature. The reaction mixture was diluted with ethyl acetate. The diluted solution was washed with 1N hydrochloric acid, an aqueous solution of sodium bicarbonate, washed with water and a saturated aqueous solution of sodium chloride, successively, dried over magnesium sulfate and concentrated to give the title compound having the following physical data.
TLC: Rf 0.35 (hexane: ethyl acetate =1:1);
NMR (300 MHz, CDCl₃): δ 7.87-7.58, 7.55-7.39, 7.27, 7.02, 6.80-6.68, 6.64-6.55, 5.50, 5.05, 3.59, 3.06, 2.70, 1.70.

### Example 7

### 3-(4-(2,5-difluorophenoxymethyl)-2-((((1R)-1-(naphthalen-2-yl)ethyl)amino)carbonyl)phenyl)propanoic acid:

To a solution of the compound prepared in Example 6 in methanol (1 mL) /THE (1 mL), 1N aqueous solution of sodium hydroxide (1 mL) was added, and the mixture was stirred overnight at room temperature. The reaction mixture was acidified by adding 1N hydrochloric acid, and extracted with ethyl acetate. The organic layer was washed with water and a saturated aqueous solution of sodium chloride, dried over magnesium sulfate and concentrated. The residue was re-crystallized with ethyl acetate / hexane to give the title compound (83 mg) having the following physical data. TLC: Rf0.47 (chloroform: methanol = 10: 1);
NMR (300 MHz, CDCl₃): δ 1.70, 2.76, 3.07, 5.05, 5.49, 6.59, 6.72, 7.02, 7.30, 7.49, 7.83.

### Example 7(1) to 7(11)

The following compounds were obtained by the same procedure as a series of reactions of Example 1 → Example 2 → Example 3 → Example 4 → Example 5 → Example 6 → Example 7 using corresponding compounds.

### Example 7(1)

3-(2-((((1R)-1-(3,5-dimethylphenyl)-3-methylbutyl)amino)carbonyl)-4-(5-fluoro-2-methylphenoxymethyl)phenyl)propanoic acid:
TLC: Rf 0.57 (chloroform: methanol = 9: 1);
NMR (300 MHz, DMSO-d₆): δ 0.90, 0.92, 1.39, 1.70, 2.13, 2.24, 2.42, 2.81, 4.97, 5.09, 6.66, 6.83, 6.92, 6.97, 7.15, 7.31, 7.38.

### Example 7(2)

3-(2-(((4-(benzothiophen-2-yl)tetrahydro-2H-pyran-4-yl)amino)carbonyl)-4-(2,5-difluorophenoxymethyl)phenyl)propanoic acid:
TLC: Rf 0.48 (chloroform: methanol = 10: 1);
NMR (300 MHz, DMSO-d₆): δ 2.06, 2.58, 2.91, 3.77, 5.22, 6.78, 7.31, 7.46, 7.77, 7.86, 8.90.

### Example 7(3)

3-(4-(2-fluoro-5-methylphenoxymethyl)-2-((((1R)-3-methyl-1-(3-methylphenyl)butyl)amino)carbonyl)phenyl)propanoic acid:
TLC: Rf 0.41 (chloroform: methanol =10: 1);
NMR (300 MHz, CDCl₃): δ 0.99, 1.74, 2.29, 2.35, 2.72, 3.03, 5.07, 5.20, 6.35, 6.74, 6.81, 6.97, 7.23, 7.42.

### Example 7(4)

3-(4-(2,5-difluorophenoxymethyl)-2-(((4-(2-(3-fluorophenyl)ethyl)tetrahydro-2H-pyran-4-yl)amino)carbonyl)phenyl)propanoic acid:
TLC: Rf 0.50 (chloroform: methanol = 9: 1);
NMR (300 MHz, CDCl₃): δ 1.80, 2.27, 2.67, 2.85, 3.13, 3.69, 3.85, 5.09, 6.11, 6.62, 6.75, 6.98, 7.21, 7.33, 7.46.

### Example 7(5)

3-(4-(2,5-difluorophenoxymethyl)-2-(((4-(2-phenylethyl)tetrahydro-2H-pyran-4-yl)amino)carbonyl)phenyl)propanoic acid:
TLC: Rf0.49 (chloroform: methanol = 10: 1);
NMR (300 MHz, CDCl₃): δ 1.53, 2.05, 226, 2.57, 2.97, 3.63, 5.20, 6.78, 7.22, 7.35, 7. 43, 8.06.

### Example 7(6)

3-(2-((((1R)-1-(3,5-dimethylphenyl)-3-methylbutyl)amino)carbonyl)-4-(3-pyridyloxymethyl)phenyl)propanoic acid:
TLC: Rf 0.49 (chloroform: methanol = 9: 1);
NMR (300 MHz, DMSO-d₆): δ 0.91, 1.42, 1.71, 2.24, 2.43, 2.83, 4.96, 5.17, 6.84, 6.95, 7.39, 8.17, 8.35, 8.76, 12.08.

### Example 7(7)

3-(4-(2-fluoro-5-methylphenoxymethyl)-2-((((1R)-1-(3,5-dimethylphenyl)-3-methylbutyl)amino)carbonyl)phenyl)propanoic acid:
TLC: Rf 0.59 (hexane: ethyl acetate = 1: 1, 1% acetic acid);
NMR (300 MHz, CDCl₃): δ 0.98, 0.99, 1.72 2.29, 2.31, 2.72, 3.03, 5.07, 5.16, 6.31, 6.72, 6.81, 6.91, 6.97, 7.28, 7.42.

### Example 7(8)

3-(4-(6-fluoroindol-1-ylmethyl)-2-((((1R)-1-(3,5-dimethylphenyl)-3-methylbutyl)amino)carbonyl)phenyl)propanoic acid:
TLC: Rf0.44 (hexane: ethyl acetate =1; 1, 1% acetic acid);
NMR (300 MHz, CDCl₃): δ 0.93, 1.60, 2.28, 2.65, 2.95, 5.08, 5.22, 6.14, 6.53, 6.88, 7.02, 7.08 7.16, 7.55.

### Example 7(9)

3-(4-(2-chloro-5-methylphenoxymethyl)-2-((((1R)-3-methyl-1-(3-methylphenyl)butyl)amino)carbonyl)phenyl)propanoic acid;
TLC: Rf 0.31 (hexane: ethyl acetate = 1: 1);
NMR (300 MHz, CDCl₃): δ 0.98, 0.99, 1.75 2.31, 2.35, 2.72, 3.04, 5.09, 5.20, 6.37, 6.75, 6.79, 7.08, 7.15, 7.26, 7.42, 7.53.

### Example 7(10)

3-(4-(2,5-difluorophenoxymethyl)-2-(((4-(2-(4-fluorophenyl)ethyl)tetrahydro-2H-pyran-4-yl)amino)carbonyl)phenyl)propanoic acid:
NMR (300 MHz, CDCl₃): δ 1.82, 2.25, 2.64, 2.84, 3.13, 3.68, 3.83, 5.08, 6.09, 6.62, 6.75, 6.99, 7.15, 7.32, 7.45.

### Example 7(11)

3-(4-(2-fluoro-5-methylphenoxymethyl)-2-(((4-(naphthalen-2-yl)tetrahydro-2H-pyran-4-yl)amino)carbonyl)phenyl)propanoic acid:
TLC: Rf 0.41 (chloroform: methanol = 10: 1);
NMR (300 MHz, CDCl₃); δ 2.30, 2.38, 2.61, 2.68 2.99, 3.91, 5.09, 6.68, 6.83, 6.98, 7.27 7.51, 7.87.

### Example 8

### 7-methoxymethoxycoumarine:

Under an atmosphere of argon, to a solution of 7-hydroxycoumarine (100 g), isopropylethylamine (161 mL) in anhydrous DMF (500 mL), methoxymethyl chloride (70.3 mL) was added dropwise at 0°C. The mixture was stirred for 4 hours at room temperature. To the reaction mixture, a mixture of hexane / ethyl acetate (2/1), and a saturated aqueous solution of sodium bicarbonate were added, and extracted with ethyl acetate. The organic layer was washed with water and a saturated aqueous solution of sodium chloride, dried over anhydrous magnesium sulfate and concentrated to give the title compound (74.1 g) having the following physical data.
TLC: Rf 0.50 (hexane: ethyl acetate = 3: 2);
NNIR (300MHz, CDCl₃): δ 7.64, 7.39, 7.01, 6.96, 6.28, 5.24, 3.49.

### Example 9

### 3-(4-methoxymethoxy-2-hydroxyphenyl)propenoic acid methyl ester:

The title compound (100 g) having the following physical data was obtained, using the compound prepared in Example 8 (74.1 g), by the same procedure as a series of reactions of Example 3.
TLC: Rf0.38 (hexane: ethyl acetate = 2: 1);
NMR (300 MHz, CDCl₃): δ 7.92, 7.39, 6.62, 6.54, 6.51, 6.01, 5.17, 3.81, 3.47.

### Example 10

### 3-(4-methoxymethaxy-2-hydroxyphenyl)propanoic acid methyl ester:

A solution of the compound prepared in Example 9 (90 g) and 10% palladium carbon (8.4 g) in methanol (1000 mL) was stirred for 7 hours at room temperature under an atmosphere of hydrogen gas. The reaction mixture was filtered through celite (trade mark). The filtrate was concentrated to give the title compound (92.1 g) having the following physical data.
TLC: Rf 0.47 (hexane: ethyl acetate = 3:2);
NMR (300 MHz, CDCl₃): δ 7.24, 6.97, 6.61, 6.57, 5.13, 3.69, 3.46, 2.84, 2.69.

### Example 11

### 3-(4-anethoxynnethoxy-2-carboxyphenyl)propanoic acid methyl ester:

The title compound (51.4 g) having the following physical data was obtained, using the compound prepared in Example 10 (82.8 g), by the same procedure as a series of reactions of Example 5.
TLC: Rf 0.34 (hexane: ethyl acetate = 1: 1);
NMR (300 MHz, CDCl₃): δ 7.71, 7.24, 7.17, 5.20, 3.67, 3.49, 3.27, 2.68.

### Example 12

### 3-(2-((((1R)-1-(naphthalen-1-yl)ethyl)amino)carbonyl)-4-methoxymethoxyphenyl)propanoic acid methyl ester:

The title compound (15.9 g) having the following physical data was obtained, using the compound prepared in Example 11 (10 g), by the same procedure as a series of reactions of Example 6.
TLC: Rf 0.69 (hexane: ethyl acetate = 1: 1);
NMR (300 MHz, CDCl₃): δ 8.23, 7.92-7.78, 7,63-7.43, 7.12, 7.03-6.95, 6.45, 6.12, 5.10, 3.61, 3.41, 3.00, 2.74-2.56, 1.80.

### Example 13

### 3-(2-((((1R)-1-(naphthalen-1-yl)ethyl)amino)carbonyl)-4-methoxymethoxyphenyl)propanol:

Under an atmosphere of argon, to a solution of the compound prepared in Example 12 (15.9 g) in anhydrous tetrahydrofuran (100 mL), lithium borohydride (2.03 g) was added in ice-bath. The mixture was stirred for 4 hours at 60°C. To the reaction mixture, water and a saturated ammonium chloride solution were added. The mixture was extracted with ethyl acetate. The organic layer was washed with water and a saturated aqueous solution of sodium chloride, dried over anhydrous magnesium sulfate and concentrated to give the title compound (13.2 g) having the following physical data.
TLC: Rf 0.32 (hexane: ethyl acetate = 1: 1);
NMR (300 MHz, CDCl₃): δ 8.21, 7.91-7.80, 7.63-7.43, 7.15, 7.02, 6.91, 6.23-6.04, 5.09, 3.59-3.43, 3.41, 2.88-2.71, 1.92-1.82, 1.79.

### Example 14

### 3-(2-((((1R)-1-(naphthalen-1-yl)ethyl)amino)carbonyl)-4-methoxymethoxyphenyl)propyl methanesulfonate:

Under an atmosphere of argon, to a solution of the compound prepared in Example 13 (13.2 g) and triethylamine (7.80 mL) in anhydrous THF (80 mL), mesylchloride (3.18 mL) was dropped in ice-bath. The mixture was stirred for 10 minutes. The reaction mixture was diluted with ethyl acetate. The diluted solution was washed with water and a saturated aqueous solution of sodium chloride, dried over anhydrous magnesium sulfate and concentrated to give the title compound (22.3 g) having the following physical data.
TLC: Rf 0.50 (hexane: ethyl acetate = 1: 1).

### Example 15

### 4-(2-((((1R)-1-(naphthalen-1-yl)ethyl)amino)carbonyl)-4-methoxymethoxyphenyl)butanonitrile:

Under an atmosphere of argon, a solution of the compound prepared in Example 14 (22.3 g) and sodium cyanide (2.01 g) in anhydrous dimethylsulfoxide (100 mL) was stirred for 5 hours at 80°C. To the reaction mixture, water was added in ice-bath. The mixture was extracted with ethyl acetate. The organic layer was washed with water and a saturated aqueous solution of sodium chloride, dried over anhydrous magnesium sulfate and concentrated. The residue was crystallized with t-butyl methyl ether / hexane to give the title compound (10.2 g) having the following physical data. TLC: Rf 0.32 (hexane: ethyl acetate= 1: 1);
NMR (300 MHz, CDCl₃): δ 8.22, 7.92-7.80, 7.64-7.44, 7.12, 7.01, 6.95, 6.18-5.99, 5.11, 3.42, 2.79, 2.30-2.12, 2.00-1.86, 1.80.

### Example 16

### 4-(2-((((1R)-1-(naphthalen-1-yl)ethyl)amino)carbonyl)-4-methoxymethoxyphenyl)butanoic acid:

To a solution of the compound prepared in Example 15 (7.13 g) in ethanol (30 mL), 33% aqueous solution of potassium hydroxide (10 mL) was added. The mixture was stirred overnight at 80°C. To the reaction mixture, 6N hydrochloric acid was added, and the mixture was extracted with ethyl acetate. The organic layer was washed with water and a saturated aqueous solution of sodium chloride, dried over anhydrous magnesium sulfate and concentrated to give the title compound (9.39 g) having the following physical data.
TLC: Rf0.33 (chloroform: methanol = 19: 1);
NMR (300 MHz, CDCl₃): δ 8.23, 7.91-7.78, 7.63-7.42, 7.11, 6.98, 6.92, 6.19-6.02, 5.08, 3.41, 2.83-2.64, 2.37-2.17, 1.97-1.82, 1.78.

### Example 17

### 4-(2-((((1R)-1-(naphthalen-1-yl)ethyl)amino)carbonyl)-4-hydroxyphenyl)butanoic acid methyl ester:

To a solution of the compound prepared in Example 16 (9.39 g) in methanol (40 mL), concentrated sulfuric acid (1 mL) was added. The mixture was stirred overnight at 60°C. To the reaction mixture, water was added in ice-bath, and the mixture was extracted with ethyl acetate. The organic layer was washed with water and a saturated aqueous solution of sodium chloride, dried over anhydrous magnesium sulfate and concentrated. The residue was crystallized with hexane to give the title compound (6.45 g) having the following physical data.
TLC: Rf 0.45 (hexane: ethyl acetate =1: 1);
NMR (300 MHz, CDCl₃): δ 8.20, 7.90-7.76, 7.59-7.40, 6.96, 6.74-6.66, 6.27-6.04, 3.57, 2.75-2.54, 2.28-2.07, 1.87-1.67, 1.76.

### Example 18

### 2-bromo-5-nitrobenzoic acid benzyl ester:

Under an atmosphere of argon, to a solution of 2-bromo-5-nitrobenzoic acid (5.14 g) in DMF, benzyl bromide (2.73 mL) and potassium carbonate (4.33 g) were added. The mixture was stirred for 30 minutes at room temperature. To the reaction mixture, water was added, and the mixture was extracted with ethyl acetate. The organic layer was washed with water and a saturated aqueous solution of sodium chloride, dried over magnesium sulfate and concentrated. The residue was washed with hexane / ethyl acetate to give the title compound (6.59 g) having the following physical data.
TLC: Rf 0.62 (hexane: ethyl acetate = 4: 1);
NMR (300 MHz, CDCl₃). δ 8.64, 8.16, 7.86, 7.49-7.39, 5.42.

### Example 19

### 2-(2-ethoxycarbonylethenyl)-5-nitrobenzoic acid benzyl ester:

Under an atmosphere of argon, to a solution of the compound prepared in Example 18 (5.57 g) in dimethyl sulfoxide (30 mL), acrylic acid ethyl ester (3.6 mL), palladium acetate (II) (186 mg), 1,1'-bis(diphenylphosphino) ferrocene (460 mg) and triethylamine (11.6 mL) were added. The mixture was stirred for 4 hours at 80°C. To the reaction mixture, ethyl acetate and water were added, and the mixture was filtered through celite (trade mark). The filtrate was extracted with ethyl acetate. The organic layer was washed with water and a saturated aqueous solution of sodium chloride, dried over magnesium sulfate and concentrated. The residue was purified by column chromatography on silica gel to give the title compound (5.33 g) having the following physical data.
TLC: Rf 0.45 (hexane: ethyl acetate = 6: 1);
NMR (300 MHz, CDCl₃): δ 8.82, 8.45, 8.36, 7.75, 7.49-7.39, 6.38, 5.42, 4.29, 1.35.

### Example 20

### 2-(2-ethoxycarbonylethyl)-5-aminobenzoic acid:

To a solution of the compound prepared in Example 19 (5.33 g) in ethanol (40 mL) / ethyl acetate (10 mL), 10% palladium carbon (500 mg) was added. The mixture was stirred for 4 hours at room temperature under an atmosphere of hydrogen gas. The reaction mixture was filtered through celite (trade mark). The filtrate was concentrated. The residue was washed with hexane / ethyl acetate to give the title compound (2.72 g) having the following physical data.
TLC: Rf 0.70 (ethyl acetate);
NMR (300 MHz, CDCl₃): δ 7.35, 7.10, 6.81, 4.11, 3.20, 2.64, 1.23.

### Example 21

### 2-(2-ethoxycarbonylethyl)-5-(t-butoxycarbonylamino)benzoic acid:

A solution of the compound prepared in Example 20 (4.00 g) and t-butyl dicarbonate (5.52 g) in THF (8mL) was stirred for 2 hours at 65°C. The reaction mixture was concentrated. The residue was crystallized with t-butyl methyl ether /hexane to give the title compound (4.86 g) having the following physical data.
TLC: Rf 0.38 (hexane: ethyl acetate =1:2);
NMR (300 MHz, CDCl₃): δ 7.94, 7.58, 7.25, 6.63, 4.12, 3.26, 2.67, 1.53, 1.23.

### Example 22

### 4-(4-(1,3-dioxaindan-5-yloxy)-2-((((1R)-1-(naphthalen-1-yl)ethyl)amino)carbonyl)phenyl)butanoic acid methyl ester:

A solution of the compound prepared in Example 17 (250 mg), 3,4-(methylenedioxy)phenylboronic acid (318 mg), copper acetate (II) (116 mg), triethylamine (445 µL) and 4Å molecular sieves (63 mg) in anhydrous methylene chloride was stirred for 3 days at room temperature. The reaction mixture was filtered through celite (trade mark), and the filtrate was diluted with ethyl acetate. The diluted solution was washed with a saturated ammonium chloride solution, water and a saturated aqueous solution of sodium chloride, successively, dried over anhydrous magnesium sulfate and concentrated. The residue was purified by column chromatography on silica gel (hexane: ethyl acetate = 3: 1) to give the title compound (85 mg) having the following physical data.
TLC: Rf 0.44 (hexane: ethyl acetate = 2: 1);
NMR (300 MHz, CDCl₃): δ 8.20, 7.91-7.79, 7.59-7.42, 7.12, 6.90-6.83, 6.72, 6.50, 6.41, 6.16-6.02, 5.99-5.95, 3.62, 2.84-2.63, 2.33-2.12, 1.96-1.83, 1.78.

### Example 23

### 4-(4-(1,3-dioxaindan-5-yloxy)-2-((((1R)-1-(naphthalen-1-yl)ethyl)amino)carbonyl)phenyl)butanoic acid:

The title compound (74 mg) having the following physical data was obtained, using the compound prepared in Example 22 (80 mg), by the same procedure as a series of reactions of Example 7.
TLC: Rf 0.56 (chloroform: methanol = 9: 1);
NMR (300 MHz, CDCl₃): δ 1.77, 1.89, 2.28, 2.75, 5.96, 6.07, 6.40, 6.49, 6.71, 6.85, 7.12, 7.51, 7.84, 8.18.

### Example 23(1) to 23(8)

The following compounds were obtained by the same procedure as a series of reactions of Example 12 → Example 17 → Example 22 → Example 23, using the compound prepared in Example 11 and a corresponding compound, or by the same procedure as a series of reactions of Example 12 → Example 22 → Example 23, using the compound prepared in Example 20 and a corresponding compound, or by the same procedure as a series of reactions of Example 12 → Example 13 → Example 14 → Example 15 → Example 16 → Example 17 → Example 22 → Example 23, using the compound prepared in Example 11 or Example 21 and a corresponding compound.

### Example 23 (1)

3-(4-(3,5-dimethylphenylamino)-2-(((4-(naphthalen-2-yl)tetrahydro-2H-pyran-4-yl)amino)carbonyl)phenyl)propanoic acid:
TLC: Rf 0.49 (chloroform: methanol = 10: 1);
NMR (300 MHz, DMSO-d₆): δ 2.05, 2.21, 2.47, 2.76, 3.80, 6.50, 6.77, 6.99, 7.13, 7.18, 7.48, 7.64, 7.87, 8.13, 8.71, 12.06.

### Example 23(2)

3-(2-(((4-(benzothiophen-2-yl)tetrahydro-2H-pyran-4-yl)amino)carbonyl)-4-(3,5-dimethylphenoxy)phenyl)propanoic acid:
TLC: Rf 0.49 (chloroform: methanol = 10: 1);
NMR (300 MHz, DMSO-d₆): δ 2.05, 2.25, 2.54, 2.87, 3.73, 6.68, 6.82, 6.97, 7.32, 7.75, 7.84, 8.85.

### Example 23(3)

3-(4-(3,5-dimethylphenylamino)-2-((((1R)-1-(3,5-dimethylphenyl)-3-methylbutyl)amino)carbonyl)phenyl)propanoic acid:
TLC: Rf 0.37 (chloroform: methanol = 19: 1);
NMR (300 MHz, CDCl₃): δ 0.96, 0.97, 1.68, 2.26, 2.29, 2.70, 2.95, 5.14, 6.23, 6.61, 6.67, 6.89, 6.93, 7.02, 7.12.

### Example 23(4)

3-(4-(3,5-dimethylphenoxy)-2-(((4-(naphthalen-2-yl)tetrahydro-2H-pyran-4-yl)amino)carbonyl)phenyl)propanoic acid:
TLC: Rf 0.45 (chloroform: methanol = 10: 1);
NMR (300 MHz, CDCl₃): δ 2.29, 2.38, 2.58, 2.67 2.96, 3.83, 3.95, 6.55, 6.63, 6.79 6.95, 7.11, 7.19, 7.46, 7.59, 7.82, 7.91.

### Example 23(5)

4-(4-(3,5-dimethylphenylamino)-2-((((1R)-1-(naphthalen-1-yl)ethyl)amino)carbonyl)phenyl)butanoic acid:
TLC: Rf 0.52 (chloroform: methanol = 9: 1);
NMR (300 MHz, CDCl₃): δ 1.76, 1.90, 2.22, 2.29, 2.72, 6.07, 6.57, 6.60, 6.91, 6.99, 7.07, 7.51, 7.84, 8.22.

### Example 23(6)

3-(4-(3,5-dimethylphenylamino)-2-((((1R)-1-(3-methylphenyl)-3-methylbutyl)amino)carbonyl)phenyl)propanoic acid:
TLC: Rf 0.56 (chloroform: methanol = 9: 1);
NMR (300 MHz, DMSO-d₆): δ 0.91, 1.38, 1.70, 2.19, 2.27, 2.41, 2.74, 4.98, 6.46 6.71, 6.98, 7.13, 8.07, 8.74.

### Example 23 (7)

3-(4-(3,5-dimethylphenoxy)-2-((((1R)-1-(3,5-dimethylphenyl)-3-methylbutyl)amino)carbonyl)phenyl)propanoic acid:
TLC: Rf0.47 (hexane: ethyl acetate = 1: 1, 1% acetic acid);
NMR (300 MHz, CDCl₃): δ 0.95, 0.96, 1.67 2.28, 2.29, 2.72, 3.00, 5.13, 6.19, 6.61, 6.77, 6.93, 7.00, 7.20.

### Example 23(8)

3-(4-(3,5-dimethylphenylamino)-2-(((4-(2-(3-fluorophenyl)ethyl)tetrahydro-2H-pyran-4-yl)amino)carbonyl)phenyl)propanoic acid:
TLC: Rf 0.52 (chloroform: methanol = 9: 1);
NMR (300 MHz, CDCl₃): δ 1.77, 2.24, 2.61, 2.82, 3.04, 3.65, 3.81, 5.99, 6.62, 6.70, 7.05.

By the example shown below, the useful of EP₃ antagonist as a preventive and/or therapeutic agent for psychoneurotic disorder can be proved. However, the present invention is not construed as being restricted thereto.

The compound (I): 3-(2-((((1R)-1-(3,5-dimethylphenyl)-3-methylbutyl)amino)carbonyl)-4-(5-fluoro-2-methylphenoxymethyl)phenyl)propanoic acid, the compound (II): 3-(2-(((1R)-3-methyl-1-(3,5-dimethylphenyl)butyl)carbamoyl)-4-(2,5-difluorophenoxymethyl)phenyl)propanoic acid, or the compound (III): 3-(2-(((1R)-3-methyl-1-(3,5-dimethylphenyl)butyl)carbamoyl)-4-(2,5-dimethylphenoxymethyl)phenyl)propanoic acid as an EP₃ antagonist are used in research.

### Example 24

### Inhibitory activity for corticosterone elevation evoked by administration of sulprostone:

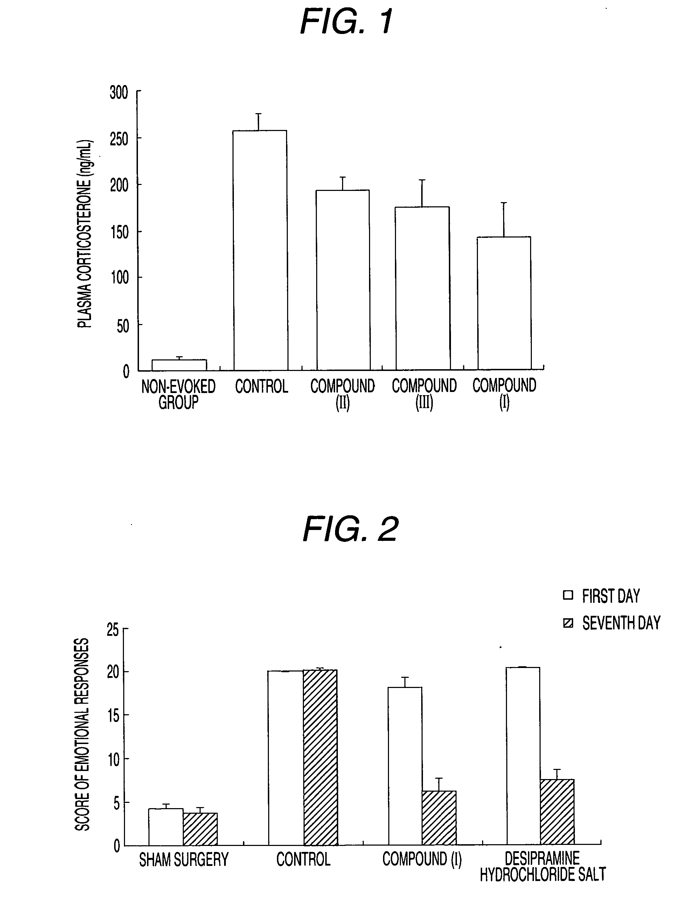

Male CD (SD) IGS rat were orally administrated EP₃ antagonist [compound (I), compound (II) and compound (III) (respectively, 10 mg/5 mL/kg (0.5% methylcellulose suspension))], the control group were orally administrated 0.5% methylcellulose suspension (5 mL/kg). After being administered for an hour, all groups were administered sulprostone subcutaneously (0.3 mg/4 mL/kg). In contrast, non-evoked group were orally administrated 0.5% methylcellulose suspension (5 mL/kg), and after being administered for an hour, administered physiological saline subcutaneously (4 mL/kg). After 1 hour from subcutaneous administration, rats of all groups were collected blood. The withdrawn blood was added EDTA and aprotinin, centrifuged (12,000 rpm, 3 minutes), and measured the corticosterone concentration in the supernatant by using the method of EIA (Bachem Inc.) or ELISA (Endocrine Technologies Inc.). The result is shown in Figure 1.

This result indicates that each corticosterone elevation was inhibited significantly by administration of the compound of the present invention.

### Example 25

### Antidepressant effect and antianxiety effect for olfactory bulbectomy rat:

Male Wistar rat was anesthetized by injection of pentobarbital sodium, and fixed the head on a brain positioner. Either side of the bulbus olfactorius region of the head was drilled a hole with dental drill after the scalp incision. The either side of the bulbus olfactorius was aspirated and removed with an aspirator and then, the head was stitched up the scalp. The rat was defined as an olfactory bulbectomy animal. Alternatively, a sham surgery animal was anesthetized, fixed the brain, incised the scalp as well as the olfactory bulbectomy animal. The either side of the bulbus olfactorius region of the head was drilled a hole, and the either side of the bulbus olfactorius was not aspirated and removed and then, the head was stitched up the scalp. The olfactory bulbectomy animals were bred with isolated alone. In contrast, the sham surgery animals were not bred with isolated alone.

The olfactory bulbectomy animals were orally administered each the compound (I) of the present invention (10 mg/kg), the compound as positive control (desipramine hydrochloride (10 mg/kg); Wako Pure Chemical Industries, Ltd.), and control (0.5% methylcellulose suspension (5 mL/kg)) twice a day for 7 days. At that time, reactivity of emotional responses for 10 minutes for the first dosage one hour later of the first day and the seventh day was marked in accordance with an evaluation standard based on Gomi's method (Gomitami, other four people, Japanese pharmacologic magazine, 1983, Vol. 82, p267-292). The result is shown in Figure 2.

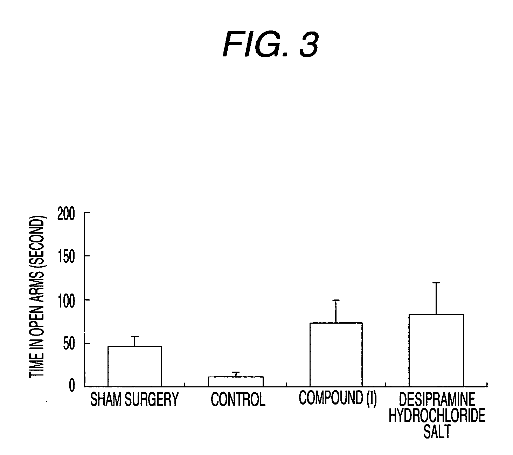

Furthermore, each the olfactory bulbectomy animals and the sham surgery animals administered the compound (I) of the present invention, desipramine hydrochloride or control were located the center of platform toward the open arms on the elevated plus-maze for 10 minutes for the first dosage one hour later of the eighth day, and it was measured within a 5 minutes period. Each the time (second) in the open arms and the close arms were measured with video-imaging behavioral analysis equipment. The result is shown in Figure 3.

It recognized that the significantly degradation score of emotional responses (seventh day: the sham surgery group; 3.7±3.4, the control group; 20.1±0.7, the compound (I) administration group; 6.2±4.6, the compound as positive control administration group; 7.5±3.4) by administration of the compound (I) and the effect is the same of more than that of desipramine hydrochloride as positive control. And so it showed that the compound (I) of the present invention has antidepressant effect. Furthermore, it recognized the significantly improvement of reduction of the time in the open arms on the elevated plus-maze. And so it showed that the compound (I) of the present invention has antianxiety effect.

The effects are not to be construed as limiting the compound (I), and it is also shown by the compound of the present invention. It is shown that the compound (II) and the compound (III) have especially, the equivalent effect as same as that of the compound (I).

### Formulation Example 1:

The following components were admixed in conventional method and punched out to obtain 10000 tablets each containing 10 mg of active ingredient.
3 -(2-(((4-(benzothiophen-2-yl)tetrahydro-2H-pyran-4-yl)amino)carbonyl)-4-(2,5-difluorophenoxymethyl)phenyl)propanoic acid: 100 g
Carboxymethylcellulose calcium (disintegrating agent): 20 g
Magnesium stearate (lubricating agent): 10 g
Microcrystalline cellulose: 870 g

### Formulation Example 2:

The following components were admixed in conventional method, and filtered through a dust filter. The resulting solution was placed 5 mL portions into ampoules, heat sterilized in an autoclave, and then freeze-dried to obtain 10,000 ampoules each containing 20 mg of the active ingredient.
3-(2-(((4-(benzothiophen-2-yl)tetrahydro-2H-pyran-4-yl)amino)carbonyl)-4-(2,5-difluorophenoxymethyl)phenyl)propanoic acid: 200 g
Mannitol: 20 g
Distilled water: 50 L

### Formulation Example 3:

3-(2-((((1R)-1-(3, 5-dimethylphenyl)-3-methylbutyl)amino)carbonyl)-4-(5-fluoro-2-methylphenoxymethyl)phenyl)propanoic acid (25 g), carboxymethyl cellulose calcium (20 g), magnesium stearate (10 g) and microcrystalline cellulose (945 g) were admixed according to a conventional method and punched out to obtain 10,000 tablets each containing 2.5 mg of the active ingredient.

### Formulation Example 4:

3-(2-((((1R)-1-(3,5-dimethylphenyl)-3-methylbutyl)amino)carbonyl)-4-(5-fluoro-2-methylphenoxymethyl)phenyl)propanoic acid (50 g), carboxymethyl cellulose calcium (20 g), magnesium stearate (10 g) and microcrystalline cellulose (920 g) were admixed according to a conventional method and punched out to obtain 10,000 tablets each containing 5 mg of the active ingredient.

### Formulation Example 5:

3-(2-((((1R)-1-(3,5-dimethylphenyl)-3-methylbutyl)amino)carbonyl)-4-(5-fluoro-2-methylphenoxymethyl)phenyl)propanoic acid (100 g), carboxymethyl cellulose calcium (20 g), magnesium stearate (10 g) and microcrystalline cellulose (870 g) were admixed according to a conventional method and punched out to obtain 10,000 tablets each containing 10 mg of the active ingredient.

### Formulation Example 6:

3-(2-((((1R)-1-(3,5-dimethylphenyl)-3-methylbutyl)amino)carbonyl)-4-(5-fluoro-2-methylphenoxymethyl)phenyl)propanoic acid (250 g), carboxymethyl cellulose calcium (40 g), magnesium stearate (20 g) and microcrystalline cellulose (690 g) were admixed according to a conventional method and punched out to obtain 10,000 tablets each containing 25 mg of the active ingredient.

### Formulation Example 7:

3-(2-((((1R)-1-(3,5-dimethylphenyl)-3-methylbutyl)amino)carbonyl)-4-(5-fluoro-2-methylphenoxymethyl)phenyl)propanoic acid (500 g), carboxymethyl cellulose calcium (60 g), magnesium stearate (20 g) and microcrystalline cellulose (520 g) were admixed according to a conventional method and punched out to obtain 10,000 tablets each containing 50 mg of the active ingredient.

### INDUSTRIAL APPLICABILITY

The compound of the present invention can be used as a preventive and/or therapeutic agent for psychoneurotic disorder, psychosomatic disorder, anxiety disorder, depression and disorder caused by stress.

## Claims

1. A preventive and/or therapeutic agent for psychoneurotic disorder, which comprises an EP₃ antagonist.

2. The preventive and/or therapeutic agent for psychoneurotic disorder according to claim 1, wherein the psychoneurotic disorder is a disorder caused by stress.

3. The preventive and/or therapeutic agent for psychoneurotic disorder according to claim 1, wherein the psychoneurotic disorder is a depression, an anxiety disorder or a psychosomatic disorder.

4. The preventive and/or therapeutic agent for psychoneurotic disorder according to claim 1, wherein the psychoneurotic disorder is a depression, an anxiety disorder or a psychosomatic disorder attributable to stress.

5. The preventive and/or therapeutic agent for psychoneurotic disorder according to claim 3, wherein the depression is a depressive episode, a dysthymic disorder, a cyclothymic disorder, a bipolar emotional disorder or a manic-depression disorder.

6. The preventive and/or therapeutic agent for psychoneurotic disorder according to claim 3, wherein the depression shows symptoms of one or more selected from psychomotor inhibition, anxiety, impatience, idea of belittlement, suicidal ideation, headache, insomnia, generalized fatigability, feeling of fatigue, febricula, vertigo, tinnitus, stiff shoulder, dry mouth feeling, taste abnormality, palpitatio cordis, dyspnoea, low back pain, arthralgia, reproductive hypesthesia, decreased libido, menstrual disorder, dysfunctional voiding, asitia, nausea, vomiting, abdominal pain, ulcus, abdominal discomfort and coldness of limbs.

7. The preventive and/or therapeutic agent for psychoneurotic disorder according to claim 3, wherein the anxiety disorder is one or more selected from obsessive-compulsive disorder, panic disorder, posttraumatic stress disorder, generalized anxiety disorder, mixed anxiety depressive disorder, social avoidance and distress and anthrophobia.

8. The preventive and/or therapeutic agent for psychoneurotic disorder according to claim 3, wherein the psychosomatic disorder shows symptoms of one or more selected from autonomic dystonia, climacteric disorder, hypertension, hypotension, angina, asthma, hyperventilation syndrome, irritable bowel syndrome, gastric ulcer, duodenal ulcer, headache, migraine, over active bladder, enuresis, insomnia, alopecia areata, diabetes, premenstrual syndrome, dysmenorrheal, infertility, alexithymia, alcohol dependence syndrome, anorexia nervosa, bulimia nervosa, Meniere's syndrome, cervicobrachial syndrome and indefinite complaint.

9. The preventive and/or therapeutic agent for psychoneurotic disorder according to claim 1, which further comprises one or more selected from tricyclic antidepressant, tetracyclic antidepressant, monoamine oxidaze inhibitor, serotonin and noradrenaline reuptake inhibitor, selective serotonin reuptake inhibitor, 5-HT_{1A} receptor agonists, GABA receptor agonists, dopamine receptor antagonist, psychoanaleptic, antianxiety agent, antipsychotic agent, mitochondrial benzodiazepine receptor agonists or antagonist, NK1 antagonist, σ receptor agonists, serotonin nervous system agonists, corticotropin releasing factor receptor antagonists, proton pump inhibitors, histamine H₂-receptor antagonist, M1 receptor antagonists and EP₁ antagonist in combination.

10. The preventive and/or therapeutic agent for psychoneurotic disorder according to claim 1, wherein the EP₃ antagonist is a compound represented by formula (I): 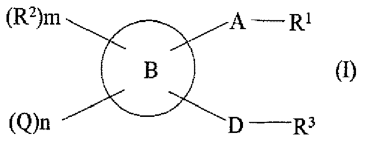 wherein R¹ is -COOH, -COOR⁴, -CH₂OH, -CONR⁵SO₂R⁶, -CONR⁷R⁸, -CH₂NR⁵SO₂R⁶, -CH₂NR⁹COR¹⁰, -CH₂NR⁹CONR⁵SO₂R⁶, -CH₂SO₂NR⁹COR¹⁰, -CH₂OCONR⁵SO₂R⁶, tetrazole, 1,2,4-oxadiazol-5-one, 1,2,4-oxadiazol-5-thione, 1,2,4-thiadiazol-5-one, 1,3-thiazolidin-2,4-dione or 1,2,3,5-oxathiadiazol-2-one;
R⁴ is C1-6 alkyl or -(C1-4 alkylene)-R¹¹;
R¹¹ is hydroxy, C1-4 alkoxy, -COOH, C1-4 alkoxycarbonyl or -CONR⁷R⁸;
R⁵ is hydrogen or C1-6 alkyl;
R⁶ is
(i) C1-6 alkyl,
(ii) a C3-15 mono-, bi- or tri-carbocyclic ring or a 3- to 15-membered mono-, bi- or tri-heterocyclic ring which is substituted with 1-5 of R¹² or unsubstituted,
(iii) C1-6 alkyl, C2-6 alkenyl or C2-6 alkynyl substituted with a C3-15 mono-, bi- or tri-carbocyclic ring or a 3- to 15-membered mono-, bi- or tri-heterocyclic ring which is substituted with 1-5 of R¹² or unsubstituted;
R⁷ and R⁸ each independently, is
(i) hydrogen,
(ii) C1-6 alkyl,
(iii) hydroxy,
(iv) -COR¹⁷,
(v) a C3-15 mono-, bi- or tri-carbocyclic ring or a 3- to 15-membered mono-, bi- or tri-heterocyclic ring which is substituted with 1-5 of R¹² or unsubstituted, or
(vi) C1-4 alkyl substituted with a C3-15 mono-, bi- or tri-carbocyclic ring or a 3-to 15-membered mono-, bi- or tri-heterocyclic ring which is substituted with 1-5 of R¹² or unsubstituted;
R⁹ is hydrogen or C1-6 alkyl;
R¹⁰ is
(i) hydrogen,
(ii) C1-6 alkyl,
(iii) a C3-15 mono-, bi- or tri-carbocyclic ring or a 3- to 15-membered mono-, bi- or tri-heterocyclic ring which is substituted with 1-5 of R¹² or unsubstituted, or
(iv) C1-6 alkyl, C2-6 alkenyl or C2-6 alkynyl substituted with a C3-15 mono-, bi- or tri-carbocyclic ring or a 3- to 15-membered mono-, bi- or tri-heterocyclic ring which is substituted with 1-5 of R¹² or unsubstituted;
R¹² is (a) C1-6 alkyl, (b) C1-6 alkoxy, (c) C1-6 alkylthio, (d) halogen, (e) CF₃, (f) cyano, (g) nitro, (h) hydroxy, (i) -COOR¹³, (j) -NHCOR¹³, (k) -SO₂R¹⁴, (1) -NR¹⁵R¹⁶, (m) a C3-7 mono-carbocyclic ring which is substituted with C1-4 alkyl or oxo or unsubstituted, (n) a 3- to 7-membered mono-heterocyclic ring which is substituted with C1-4 alkyl or oxo or unsubstituted, or (o) C1-4 alkyl substituted with hydroxy, -COOR¹³, -NHCOR¹³, -SO₂R¹⁴ or -NR¹⁵R¹⁶;
R¹³ is hydrogen, C1-4 alkyl, phenyl, or phenyl-(C1-4) alkyl;
R¹⁴ is C1-4 alkyl;
R¹⁵ and R¹⁶ each independently, is hydrogen, C1-4 alkyl, phenyl, or phenyl-(C1-4) alkyl;
R¹⁷ is C1-4 alkyl or phenyl;
A is
(i) a single bond,
(ii) C1-6 alkylene,
(iii) C2-6 alkenylene,
(iv) C2-6 alkynylene,
(v) -O-(C1-3 alkylene),
(vi) -S-(C 1-3 alkylene),
(vii) -NR²⁰-(C1-3 alkylene),
(viii) -CONR²¹-(C1-3 alkylene),
(ix) -(C1-3 alkylene)-O-(C1-3 alkylene),
(x) -(C1-3 alkylene)-S-(C1-3 alkylene),
(xi) -(C1-3 alkylene)-NR²⁰-(C1-3 alkylene),
(xii) -(C1-3 alkylene)-CONR²¹-(C1-3 alkylene),
(xiii) -Cycl,
(xiv) -(C1-4 alkylene)-Cycl or
(xv) -Cyc1-(C1-4 alkylene);
the alkylene, alkenylene and alkynylene in A may be substituted with 1-6 of the following substituents of (a)-(i):
(a) C1-6 alkyl, (b) C1-6 alkoxy, (c) halogen, (d) CHF₂, (e) CF₃, (f) OCHF₂, (g) OCF₃, (h) hydroxy, (i) hydroxy-(C1-4) alkyl;
R²⁰ is hydrogen, C1-4 alkyl, -SO₂-(C1-4) alkyl or C2-5 acyl;
R²¹ is hydrogen or C1-4 alkyl;
Cyc1 is a C3-7 mono-carbocyclic ring or a 3- to 7-membered mono-heterocyclic ring which is substituted with 1-4 of C1-6 alkyl, C1-6 alkoxy, C1-6 alkylthio, C2-6 alkenyl, C2-6 alkynyl, halogen, CHF₂, CF₃, nitro or cyano, or unsubstituted;
B ring is a C3-12 mono- or bi-carbocyclic ring or a 3- to 12-membered mono- or bi-heterocyclic ring;
R² is C1-6 alkyl, C1-6 alkoxy, C1-6 alkylthio, C2-6 alkenyl, C2-6 alkynyl, halogen, CHF₂, CF₃, nitro, cyano, phenyl or oxo;
m is 0, 1 or 2;
n is 1 or 2 when -D-R³ binds to B ring at the ortho position based on -A-R¹;
n is 0, 1 or 2 when -D-R³ binds to B ring at the non-ortho position based on -A-R¹;
Q is
(1)
(i) -(C1-4 alkylene, C2-4 alkenylene or C2-4 alkynylene)-Cyc2,
(ii) -(C1-4 alkylene)-Z-Cyc3,
(iii) C1-4 alkyl substituted with a substituent(s) selected from -NR²⁴R²⁵, -S(O)ₚR²⁵, cyano, -NR²³COR²⁷, -NR²³SO₂R²⁸ and -NR²³CONR²⁴R²⁵,
(iv) C1-4 alkoxy(C1-4) alkoxy, -NR²³COR²⁷, -COR²⁸, -OSO₂R²⁸, -NR²³SO₂R²⁸ or -NR²³CONR²⁴R²⁵,
(v) a C3-7 mono-carbocyclic ring or a 3- to 6-membered mono-heterocyclic ring which is substituted with 1-5 of R³⁰, wherein one R³⁰ of them always binds to the ring at the non 1-position,
(vi) a C8-15 mono-, bi- or tri-carbocyclic ring or a 7- to 15-membered mono-, bi- or tri-heterocyclic ring which is substituted with 1-5 of R³⁰ or unsubstituted,
(vii) -T-Cyc5,
(viii) -L-Cyc6-1, -L-(C3-6 cycloalkyl), -L-CH₂-(C3-6 cycloalkyl), -L-(C2-4 alkylene)-Cyc6-2 or -L-(C1-4 alkylene)_{q}-Cyc6-3, wherein the cycloalkyl is substituted with 1-5 of R³⁰ or unsubstituted,
(2)
(i) phenoxy,
(ii) benzyloxy,
(iii) hydroxy(C1-4) alkyl,
(iv) C1-4 alkoxy(C 1-4) alkyl, or
(v) -(C1-4 alkylene)-O-benzyl, or
(3)
(i) C2-6 alkenyl,
(ii) C2-6 alkynyl,
(iii) C1-6 alkyl substituted with 1-3 halogen(s),
(iv) cyano,
(v) nitro,
(vi) -NR³³R³⁴,
(vii) -CONR³³R³⁴,
(viii) -S(O)ₚ-(C1-4) alkynyl,
(ix) -S(O)ₚ-CHF₂,
(x) -S(O)ₚ-NR³³R³⁴,
(xi) -O-(C3-6) alkynyl,
(xii) -O-CHF₂, or
(xiii) C3-7 cycloalkyl;
R²² is hydrogen, Cl-4 alkyl, -SO₂(C1-4) alkyl or C2-5 acyl;
R²³ is hydrogen, C1-4 alkyl, phenyl or phenyl(C1-4) alkyl;
R²⁴ and R²⁵ each independently, is hydrogen, C1-4 alkyl, Cyc4 or (C1-4 alkylene)-Cyc4;
R²⁶ is C1-4 alkyl or Cyc4;
R²⁷ is hydrogen, C1-4 alkyl, -OR²⁹ or Cyc4;
R²⁸ is C1-4 alkyl, Cyc4 or -(C1-4 alkylene)-Cyc4;
R²⁹ is hydrogen, C1-4 alkyl, Cyc4 or (C1-4 alkylene)-Cyc4;
R³⁰ is C1-8 alkyl, C1-8 alkoxy, C1-8 alkylthio, halogen, CF₃, OCF₃, SCF₃, CHF₂, OCHF₂, SCHF₂, hydroxy, cyano, nitro, -NR³¹R³², -CONR³¹R³², formyl, C2-5 acyl, hydroxy(C1-4) alkyl, C1-4 alkoxy(C1-4) alkyl, C1-4 alkylthio(C1-4) alkyl, -(C1-4 alkylene)-CONR³¹R³², -SO₂(C1-4) alkyl, -NR²³CO-(C1-4) alkyl, -NR²³SO₂-(C1-4) alkyl, benzoyl, oxo, a C3-7 mono-carbocyclic ring, a 3- to 7-membered mono-heterocyclic ring, -(C1-4 alkylene)-NR³¹R³², -M-(C3-7 mono-carbocyclic ring), or -M-(3- to 7-membered mono-heterocyclic ring);
the C3-7 mono-carbocyclic ring and 3- to 7-membered mono-heterocyclic ring in R³⁰ may be substituted with 1-5 of the following substituents (a)-(1):
(a) C 1-6 alkyl, (b) C2-6 alkenyl, (c) C2-6 alkynyl, (d) C1-6 alkoxy, (e) C1-6 alkylthio, (f) halogen, (g) CHF₂, (h) CF₃, (I) nitro, (j) cyano, (k) hydroxy, (1) amino;
M is -O-, -S-, C1-4 alkylene, -O-(C1-4 alkylene)-, -S-(C1-4 alkylene)-, -(C1-4 alkylene)-O- or -(C1-4 alkylene)-S-;
R³¹ and R³² each independently, is hydrogen or C1-4 alkyl,
Cyc2 is a C3-15 mono-, bi- tri-carbocyclic ring or a 3- to 15-membered mono-, bi- tri-heterocyclic ring which is substituted with 1-5 of R³⁰ or unsubstituted;
Z is -O-, -S(O)ₚ-, -NR²²-, -NR²³CO- -NR²³SO₂-, -NR²²-(C1-4 alkylene)-, -S(O)ₚ-(C1-4 alkylene)-, -O-(C2-4 alkylene)-, -NR²³CO-(C1-4 alkylene) or -NR²³SO₂-(C1-4 alkylene);
p is 0, 1 or 2;
Cyc3 is a C3-15 mono-, bi- tri-carbocyclic ring or a 3- to 15-membered mono-, bi- tri-heterocyclic ring which is substituted with 1-5 of R³⁰ or unsubstituted;
Cyc4 is a C3-12 mono-, bi-carbocyclic ring or a 3- to 12-membered mono-, bi-heterocyclic ring which is substituted with 1-5 of R³⁰ or unsubstituted;
T is -O-, -NR²²-, -O-(C1-4 alkylene)-, -S(O)ₚ-(C1-4 alkylene)- or -NR²²-(C1-4 alkylene)-;
Cyc5 is a 3- to 15-membered mono-, bi- tri-heterocyclic ring which is substituted with 1-5 of R³⁰ or unsubstituted;
q is 0 or 1;
L is -O- or -NR²³-;
Cyc6-1 is phenyl or benzyl which is substituted with one or more R³⁰;
Cyc6-2 is a C3-6 mono-carbocyclic ring which is substituted with 1-5 of R³⁰ or unsubstituted;
Cyc6-3 is a C7-15 mono-, bi- or tri-carbocyclic ring which is substituted with 1-5 of R³⁰ or unsubstituted;
R³³ and R³⁴ each independently, is hydrogen, C1-4 alkyl, phenyl or benzyl, or
NR³³R³⁴ is a 3- to 6-membered mono-heterocyclic ring containing one nitrogen and optionally containing one hetero atom selected from nitrogen, oxygen and sulfur;
D is
(1) a 1- or 2-membered linking chain comprising an atom(s) selected from carbon, nitrogen, oxygen and sulfur, which may contain a double bond or a triple bond in the chain and may be substituted with 1-4 of R⁴⁰,
(2) a 3- to 6-membered linking chain comprising atoms selected from carbon, nitrogen, oxygen and sulfur, which may contain a double bond or a triple bond in the chain and may be substituted with 1-12 of R⁴⁰, wherein R⁴⁰ substituted on the atom bound to R³, and R⁴² which is a substituent of R³, taken together may form -(CH₂)_{y}-, in which y is 1-4, or
(3) a 7- to 10-membered linking chain comprising atoms selected from carbon, nitrogen, oxygen and sulfur, which may contain a double bond or a triple bond and may be substituted with 1-20 of R⁴⁰, wherein R⁴⁰ substituted on the atom bound to R³, and R⁴² which is a substituent of R³, taken together may form -(CH₂)_{y}-;
R⁴⁰ is (a) C1-8 alkyl, (b) C2-8 alkenyl, (c) C2-8 alkynyl, (d) oxo, (e) halogen, (f) CF₃, (g) hydroxy, (h) C1-6 alkoxy, (i) C2-6 alkenyloxy, (j) C2-6 alkynyloxy, (k) OCF₃, (1) -S(O)ₚ-(C1-6) alkyl, (m) -S(O)ₚ-(C2-6) alkenyl, (n) -S(O)ₚ-(C2-6) alkynyl, (o) C2-5 acyl, (p) Cyc9, (q) C1-4 alkoxy(C1-4) alkoxy, or (r) C1-8 alkyl, C2-8 alkenyl or C2-8 alkynyl substituted with 1 or 2 of substituents selected from halogen, CF₃, OCF₃, hydroxy, cyano, Cl-4 alkoxy, -S(O)ₚ-(C1-6) alkyl, Cyc9 and C1-4 alkoxy(C1-4) alkoxy or
two R⁴⁰'s taken together with the atom of a linking chain to which they bind, may form a C3-15 mono-, bi- or tri-carbocyclic ring or a 3- to 15-membered mono-, bi-or tri-heterocyclic ring containing 1-2 hetero atom(s) selected from O, S, SO₂ and N, wherein the carbocyclic ring and the heterocyclic ring may be substituted with 1-3 substituent(s) selected from C1-4 alkyl, C1-4 alkoxy, C2-5 acyl, SO₂(C1-4 alkyl), phenyl and phenyl(C1-4) alkyl;
Cyc9 is a C3-6 mono-carbocyclic ring or a 3- to 6-membered mono-heterocyclic ring, which is substituted with 1-5 of R⁴¹ or unsubstituted;
R⁴¹ is C1-4 alkyl, C1-4 alkoxy, C1-4 alkylthio, C1-4 alkoxy(C1-4) alkyl, halogen, CF₃, OCF₃, SCF₃, hydroxy, cyano, formyl, C2-5 acyl, -SO₂-(C1-4) alkyl, -NR²³CO-(C1-4) alkyl, benzoyl or oxo;
R³ is
(1) C1-6 alkyl or
(2) a C3-15 mono-, bi- or tri-carbocyclic ring or a 3- to 15-membered mono-, bi- or tri-heterocyclic ring which is substituted with 1-5 of R⁴² or unsubstituted;
R⁴² is (a) C1-6 alkyl, (b) C1-6 alkoxy, (c) C1-6 alkylthio, (d) halogen, (e) cyano, (f) CF₃, (g) CHF₂, (h) OCF₃, (i) OCHF₂, (j) SCF₃, (k) -NR⁴³R⁴⁴, (1) -SO₂R⁴⁵, (m) -NR⁴⁶COR⁴⁷, (n) hydroxy, (o) oxo, (p) C1-4 alkoxy(C1-4) alkyl, (q) Cyc10, (r) C1-6 alkylene-Cyc10, (s) -CO-Cyc10, (t) -W-Cyc10, (u) -(C1-6 alkylene)-W-Cyc10, (v) -W-(C1-6 alkylene)-Cyc10 or (w) -(C1-6 alkylene)-W-(C1-6 alkylene)-Cyc10;
R⁴³ and R⁴⁴ each independently, is hydrogen or C1-4 alkyl;
R⁴⁵ is C1-4 alkyl;
R⁴⁶ is hydrogen or C1-4 alkyl;
R⁴⁷ is hydrogen or C1-4 alkyl;
Cyc10 is a C3-12 mono- or bi-carbocyclic ring or a 3- to 12-membered mono- or bi-heterocyclic ring which is substituted with 1-5 of substitutes of the following (a)-(j) or unsubstituted,
(a) C1-4 alkyl, (b) C2-5 acyl, (c) 1-4 alkoxy, (d) halogen, (e) hydroxy, (f) nitro, (g) cyano, (h) amine, (i) CF₃, (j) OCF₃;
W is -O-, -S(O)ₚ- or -NR⁴⁸-;
R⁴⁸ is hydrogen or C1-4 alkyl;
or a salt thereof, a solvate thereof or a prodrug thereof.

11. The preventive and/or therapeutic agent for psychoneurotic disorder according to claim 10, which is
(1) 3-(2-(((1R)-3-methyl-1-(3,5-dimethylphenyl)butyl)carbamoyl)-4-(2,5-difluorophenoxymethyl)phenyl)propanoic acid,
(2) 3-(2-(((1R)-3-methyl-1-(3,5-dimethylphenyl)butyl)carbamoyl)-4-(2,5-dimethylphenoxymethyl)phenyl)propanoic acid,
(3) 3-(2-((((1R)-1-(3,5-dimethylphenyl)-3-methylbutyl)amino)carbonyl)-4-((2-methylphenoxy)methyl)phenyl)propanoic acid,
(4) 3-(4-((2,5-difluorophenoxy)methyl)-2-(((4-(3,5-dimethylphenyl)tetrahydro-2H-pyran-4-yl)amino)carbonyl)phenyl)propanoic acid,
(5) 3-(4-(3-cyanophenoxy)methyl)-2-((((1R)-1-(3,5-dimethylphenyl)-3-methylbutyl)amino)carbonyl)phenyl)propanoic acid,
(6) 3-(4-((3-chlorophenoxy)methyl)-2-((((1R)-1-(3,5-dimethylphenyl)-3-methylbutyl)amino)carbonyl)phenyl)propanoic acid,
(7) 3-(4-((2-chloro-5-fluorophenoxy)methyl)-2-(((4-(3,5-dimethylphenyl)tetrahydro-2H-pyran-4-yl)amino)carbonyl)phenyl)propanoic acid,
(8) 3-(4-((2-chloro-5-methylphenoxy)methyl)-2-(((4-(3,5-dimethylphenyl)tetrahydro-2H-pyran-4-yl)amino)carbonyl)phenyl)propanoic acid,
(9) 3-(4-((2,5-dichlorophenoxy)methyl)-2-(((4-(3,5-dimethylphenyl)tetrahydro-2H-pyran-4-yl)amino)carbonyl)phenyl)propanoic acid or
(10) 3-(4-((2,5-difluorophenoxy)methyl)-2-(((4-(3-methylphenyl)tetrahydro-2H-pyran-4-yl)amino)carbonyl)phenyl)propanoic acid,
a salt thereof, a solvate thereof or a prodrug thereof

12. The preventive and/or therapeutic agent for psychoneurotic disorder according to claim 1, wherein the EP₃ antagonist is a compound represented by formula (II): 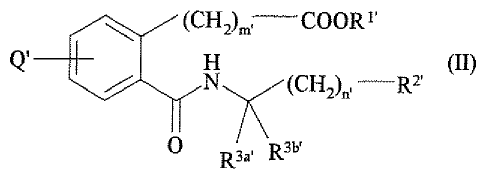 wherein R^{1'} is hydrogen or C 1-4 alkyl;
R^{2'} is phenyl, naphthyl, benzofuranyl or benzothionyl, which is unsubstituted or substituted with 1-2 of C1-4 alkyl and halogen;
Q' is (i) -CH₂-O-Cyc1', (ii) -CH₂-Cyc2' or (iii) -L'-Cyc3';
Cyc1' is phenyl or pyridyl, which is unsubstituted or substituted with 1-2 of R^{4'};
Cyc2' is indolyl which is unsubstituted or substituted with 1-2 of R^{4'};
Cyc3' is phenyl substituted with 1-2 of R^{4'};
L' is -O- or -NH-;
R^{3a'} and R^{3b'} each independently is hydrogen or C1-4 alkyl or
R^{3a'} and R^{3b'}, taken together with the carbon atom to which they are attached, form tetrahydro-2H-pyran;
m' is 2 or 3;
n' is 0, 1 or 2;
R^{4'} is C1-4 alkyl, C1-4 alkylthio, halogen or cyano, or when Cyc3' is phenyl substituted with two R^{4'}, two R^{4'} taken together with phenyl may form 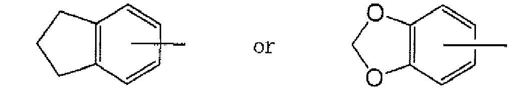 a salt thereof, a solvate thereof or a prodrug thereof.

13. The preventive and/or therapeutic agent for psychoneurotic disorder according to claim 12, which is
(1) 3-(2-((((1R)-1-(3,5-dimethylphenyl)-3-methylbutyl)amino)carbonyl)-4-(5-fluoro-2-methylphenoxymethyl)phenyl)prapanoie acid,
(2) 3-(2-(((4-(benzothiophen-2-yl)tetrahydro-2H-pyran-4-yl)amino)carbonyl)-4-(2,5-difluorophenoxymethyl)phenyl)propanoic acid,
(3) 3-(4-(2-fluoro-5-methylphenoxymethyl)-2-((((1R)-3-methyl-1-(3-methylphenyl)butyl)amino)carbonyl)phenyl)propanoic acid,
(4) 3-(4-(2,5-difluorophenoxymethyl)-2-(((4-(2-(3-fluorophenyl)ethyl)tetrahydro-2H-pyran-4-yl)amino)carbonyl)phenyl)propanoic acid,
(5) 3-(4-(3,5-dimethylphenylamino)-2-(((4-(naphthalen-2-yl)tetrahydro-2H-pyran-4-yl)amino)carbonyl)phenyl)propanoic acid,
(6) 3-(2-(((4-(benzothiophen-2-yl)tetrahydro-2H-pyran-4-yl)amino)carbonyl)-4-(3,5-dimethylphenoxy)phenyl)propanoic acid,
(7) 3-(4-(2,5-difluorophenoxymethyl)-2-(((4-(2-phenylethyl)tetrahydro-2H-pyran-4-yl)amino)carbonyl)phenyl)propanoic acid,
(8) 3-(2-((((1R)-1-(3,5-dimethylphenyl)-3-methylbutyl)amino)carbanyl)-4-(3-pyridyloxymethyl)carbonyl)phenyl)propanoic acid,
(9) 3-(4-(2-fluoro-5-methylphenoxymethyl)-2-((((1R)-1-(3,5-dimethylphenyl)-3-methylbutyl)amino)carbonyl)phenyl)propanoic acid,
(10) 3-(4-(3,5-dimethylphenylamino)-2-((((1R)-1-(3,5-dimethylphenyl)-3-methylbutyl)amino)carbonyl)phenyl)propanoic acid,
(11) 3-(4-(6-fluoroindol-1-ylmethyl)-2-((((1R)-1-(3,5-dimethylphenyl)-3-methylbutyl)amino)carbonyl)phenyl)propanoic acid,
(12) 3-(4-(3,5-dimethylphenoxy)-2-(((4-(naphthalen-2-yl)tetrahydro-2H-pyran-4-yl)amino)carbonyl)phenyl)propanoic acid,
(13) 4-(4-(3,5-dimethylphenylamino)-2-((((1R)-1-(naphthalen-1-yl)ethyl)amino)carbonyl)phenyl)butanoic acid,
(14) 3-(4-(2-chloro-5-methylphenoxymethyl)-2-((((1R)-3-methyl-1-(3-methylphenyl)butyl)amino)carbonyl)phenyl)propanoic acid,
(15) 3-(4-(2,5-difluorophenoxymethyl)-2-(((4-(2-(4-fluorophenyl)ethyl)tetrahydro-2H-pyran-4-yl)amino)carbonyl)phenyl)propanoic acid,
(16) 3-(4-(3,5-dimethylphenylamino)-2-((((1R)-1-(3-methylphenyl)-3-methylbutyl)amino)carbonyl)phenyl)propanoic acid,
(17) 3-(4-(2-fluoro-5-methylphenoxymethyl)-2-(((4-(naphthalen-2-yl)tetrahydro-2H-pyran-4-yl)amino)carbonyl)phenyl)propanoic acid,
(18) 3-(4-(3,5-dimethylphenoxy)-2-((((1R)-1-(3,5-dimethylphenyl)-3-methylbutyl)amino)carbonyl)phenyl)propanoic acid or
(19) 3-(4-(3,5-dimethylphenylamino)-2-(((4-(2-(3-fluorophenyl)ethyl)tetrahydro-2H-pyran-4-yl)amino)carbonyl)phenyl)propanoic acid,
a salt thereof, a solvate thereof or a prodrug thereof

14. The preventive and/or therapeutic agent for psychoneurotic disorder according to claim 1, which comprises the EP₃ antagonist in the range of from about 1 mg to about 600 mg.

15. The preventive and/or therapeutic agent for psychoneurotic disorder according to claim 14, wherein the EP₃ antagonist is 3-(2-((((1R)-1-(3,5-dimethylphenyl)-3-methylbutyl)amino)carbonyl)-4-(5-fluoro-2-methylphenoxymethyl)phenyl)propanoic acid, 3-(2-(((1R)-3-methyl-1-(3,5-dimethylphenyl)butyl)carbamoyl)-4-(2,5-difluorophenoxymethyl)phenyl)propanoic acid or 3-(2-(((1R)-3-methyl-1-(3,5-dimethylphenyl)butyl)carbamoyl)-4-(2,5-dimethylphenoxymethyl)phenyl)propanoic acid.

16. A preventive and/or therapeutic agent for psychoneurotic disorder, which comprises 3-(2-((((1R)-1-(3,5-dimethylphenyl)-3-methylbutyl)amino)carbonyl)-4-(5-fluoro-2-methylphenoxymethyl)phenyl)propanoic acid which is administered in the range of from about 1 mg to about 600 mg per day.

17. The preventive and/or therapeutic agent for psychoneurotic disorder according to claim 16, wherein the psychoneurotic disorder is a depression or an anxiety disorder.

18. The preventive and/or therapeutic agent for psychoneurotic disorder according to claim 16, wherein it is administered in the range of from about 5 mg to about 300 mg per day.

19. The preventive and/or therapeutic agent for psychoneurotic disorder according to claim 18, wherein it is administered in the range of from about 10 mg to about 100 mg per day.

20. The preventive and/or therapeutic agent for psychoneurotic disorder according to claim 19, wherein it is administered in the range of from about 10 mg to about 50 mg per day.

21. The preventive and/or therapeutic agent for psychoneurotic disorder according to claim 16, wherein 3-(2-((((1R)-1-(3,5-dimethylphenyl)-3-methylbutyl)amino)carbonyl)-4-(5-fluoro-2-methylphenoxymethyl)phenyl)propanoic acid is contained in the range of from about 1 mg to about 100 mg per solid preparation.

22. The preventive and/or therapeutic agent for psychoneurotic disorder according to claim 21, wherein it is contained in the range of from about 2.5 mg to about 25 mg per solid preparation.

23. A method for preventing and/or treating psychoneurotic disorder, which comprises administering an effective dose of the compound of formula (I) or formula (II), a salt thereof, a solvate thereof or a prodrug thereof to a mammal.

24. The method according to claim 23, wherein the compound of formula (II) is 3-(2-((((1R)-1-(3,5-dimethylphenyl)-3-methylbutyl)amino)carbonyl)-4-(5-fluoro-2-methylphenoxymethyl)phenyl)propanoic acid and is administered in the range of from about 1 mg to about 600 mg per day.

25. Use of a compound of formula (I) or (II), a salt thereof, a solvate thereof or a prodrug thereof, for the manufacture of a preventive and/or therapeutic agent for psychoneurotic disorder.

26. The use according to claim 25, wherein the compound of formula (II) is 3-(2-((((1R)-1-(3,5-dimethylphenyl)-3-methylbutyl)amino)carbonyl)-4-(5-fluoro-2-methylphenoxymethyl)phenyl)propanoic acid and is administered in the range of from about 1 mg to about 600 mg per day.
